(19) Europäisches Patentamt
European Patent Office
Office européen des brevets

(11) EP 0 574 510 B1

(12) **EUROPEAN PATENT SPECIFICATION**

(45) Date of publication and mention of the grant of the patent: **05.09.2001 Bulletin 2001/36**

(21) Application number: **92907890.5**

(22) Date of filing: **04.03.1992**

(51) Int Cl.[7]: **A61K 31/34**, A61K 31/36, A61K 31/385, A61K 31/38, A61K 31/44, C07D 409/08, C07D 401/08, C07D 319/18, C07D 317/54, C07D 321/10, C07D 339/06, C07D 307/77, C07D 407/06, C07D 409/12

(86) International application number: **PCT/US92/01830**

(87) International publication number: **WO 92/15294 (17.09.1992 Gazette 1992/24)**

(54) **2,4-DIARYL-1,3-DITHIOLANES AS PLATELET ACTIVATING FACTOR RECEPTOR ANTAGONISTS AND 5-LIPOXYGENASE INHIBITORS**

2,4-DIARYL-1,3-DITHIOLANE ALS PLATELET ACTIVATING FAKTOR REZEPTORBLOCKER UND 5-LIPOXYGENASEHEMMER

2,4-DIARYL-1,3-DITHIOLANES UTILISES COMME ANTAGONISTES DE RECEPTEURS DE FACTEURS D'ACTIVATION DES PLAQUETTES ET INHIBITEURS DE 5-LIPOXYGENASE

(84) Designated Contracting States: **AT BE CH DE DK ES FR GB GR IT LI LU MC NL SE**

(30) Priority: **04.03.1991 US 664553**

(43) Date of publication of application: **22.12.1993 Bulletin 1993/51**

(73) Proprietor: **CENTER FOR INNOVATIVE TECHNOLOGY**
**Herndon, VA 22070 (US)**

(72) Inventors:
- **SHEN, T.Y.**
  **Charlottesville, VA 22903 (US)**
- **GOLDSTEIN, David**
  **Charlottesville, VA 22901 (US)**
- **GINGRICH, Diane M.**
  **Charlottesville, VA 22903 (US)**

(74) Representative: **Bassett, Richard Simon et al**
**Eric Potter Clarkson,**
**Park View House,**
**58 The Ropewalk**
**Nottingham NG1 5DD (GB)**

(56) References cited:
**EP-A- 0 365 089** **US-A- 4 539 332**
**US-A- 4 987 132**

- **CHEMICAL ABSTRACTS, vol. 97, no. 7, 1982, Columbus, Ohio, US; abstract no. 52493k, B.TALAPATRA ET AL. '(-)MAGLIFLOENONE' page 346 ; & PHYTOCHEMISTRY vol. 21, no. 3 , 1982 pages 747 - 750**
- **CHEMICAL ABSTRACTS, vol. 100, no. 26, 1984, Columbus, Ohio, US; abstract no. 209491p, Y.SHISURI ET AL. 'SYNTHESIS OF SOME PHYSIOLOGICALLY ACTIVE SUBSTANCES' page 565 ; & TENNEN YUKI KAGOBUTSU TORONKAI KOEN YOSHISHU vol. 26 , 1983 , KEIO pages 437 - 444**
- **TETRAHEDRON LETTERS no. 16 , 1968 , OXFORD GB pages 2009 - 2014 M.C. WOODS,I. MIURA. 'THE NMR SPECTRA OF FUTOENONE AND DERIVATIVES:'**
- **Tetrahedron Letters, 1988, E.J. CORREY et al., "Duel Binding Modes to the Receptor for Platelet Activating Factor (PAF) of Anti-PAF trans-2,5 diayilfurans", Vol. 29 (No. 24) pp. 2899-2902, see page 2901.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 574 510 B1

- Tetrahedron Letters, 1968, A. OGISO et al., "The Structure of Futoenone, a Novel Spircyclohexadienone Derivative", No. 16, pp. 2003-2008, see page 2005.
- Chemical and Pharmaceutical Bulletin, 1970, A. OGISO et al., "The Structure and Total Synthesis of Futoenone, a Constiuent of Piper futokadzura Sieb et Zucc.", Vol. 18, No. 1, pp. 105-114, see p. 107.

## Description

## BACKGROUND OF THE INVENTION

Field of the Invention

The present invention is generally related to 2,4-diaryl-1,3-dithiolanes which inhibit both platelet activating factor and 5-lipoxygenase for the treatment of inflammatory and allergic disorders.

Description of the Prior Art

Platelet activating factor (PAF) is a highly potent ether linked phospholipid (1-0-alkyl-2-acetyl-sn-glycerol-3-phosphorylcholine) which activates platelets as well as modulates the function of leukocytes and other target cells. PAF has been shown to be a mediator of a variety of pathophysiological conditions including arthritis, acute inflamation, asthma, endotoxic shock, pain, psoriasis, ophthalmic inflammation, ischemia, and gastrointestinal ulceration. Interaction with a specific membrane recognition site coupled to phosphatidylinositol metabolism produces the biological activity of PAF. Hence, blocking the FAF receptor can provide beneficial medical results in human beings and mammals suffering from diseases or disorders mediated by PAF.

Several PAF antagonists have recently been synthesized or isolated from natural sources. For example, Shen et al. in Proc. Natl. Acad. Sci. (U.S.A.), 82. 672-678 (1985), reported that kadsurenone, a neoliguan derivative isolated from Piper fotukadsura Sieb et Zucc (a Chinese herbal plant), was a potent, specific and competitive inhibitor of PAF at the receptor level. Biftu et al., in J. Med. Chem. 29, 1917 (1986), and Ponpipom et al., in Biochem. Bioshys.

Res. Comm. 150, 1213 (1988), have shown that 2,5-diaryltetrahydrofurans L-652,731 and L-659,989 (both of which are synthetic analogs of neolignan), respectively, are potent PAF receptor antagonists. U.S. Patent 4,539,332 to Biftu et al. and U.S. Patent 4,595,693 to Biftu et al. are both related to the use of 2,5-diaryltetrahydrofurans and their analogs as PAF antagonists. Graham et al., in 197th Amer. Chem. Soc. National Meeting Abstracts, 1989, MEDI, 25, 20, Corey, et al. in Tet. Lett., Vol. 29, 2899-2902 (1988), and U.S. Patent 4,656,190 to Shen et al. respectively show that 1,3-diarylcyclopentanes, 2,4-diaryldioxolanes, and indene derivatives are also potent PAF receptor antagonists.

Leukotrienes, lke PAF, are potent lipid mediators of a variety of topical and systemic diseases and disorders. 5-lipoxygenase catalyzes the conversion of arachidonic acid to leukotriene A4 which is the precursor of leukotrienes B4 and C4. Leukotrienes B4 and C4 are oxygenated metabolites that contribute to the pathogenesis of such inflammatory disorders as arthritis, asthma, psoriasis, and thrombotic disease. Leukotrienes are released concomitantly from leukocytes with PAF from a common phospholipid precursor upon cellular activation and act synergistically with PAF in many biological models. It has been demonstrated that a physical combination of a PAF antagonist and a leukotriene inhibitor is significantly more effective than either agent alone in treating asthma in an animal model (see, O'Donnell et al. in Therapeutic Approaches to inflammatory Diseases, Lewis et al., Elsevier, New York, 1989, pp.169-193).

Shen et al., in PAF and Related Lipid Mediators, Plenum Pub., N.Y., 164 (1987) and Page et al., in Trends in Pharmacol. Sci. 10, 1 (1989), pointed out that single compounds which posess the dual inhibitory capability of PAF and leukotriene inhibition would have greater antiinflammatory activities than a physical combination of a PAF and a leukotriene inhibitor. Moreover, the chemical combination of PAF and 5-lipoxygenase inhibitory activities in one molecule has advantages over drug combinations in terms of optimal pharmacokinetics, clinical applications and developmental costs. However, few compounds are known which posess this dual inhibitory activity. Shen et al., in PAF and Related Lipid Mediators, Plenum Pub: N.Y., 153-190 (1987), reported that a tetrahydrothiophene analog of lignan, L-653,150, is both a potent PAF antagonists and a moderate inhibitor of 5-lipoxygenase. European Patent Application 0,365,089 to Biftu, filed October 13, 1989, is also directed the use of tetrahydrothiophene analoqs as leukotriene inhibitors and specific PAF antagonists.

Because of the large number of diseases and disorders which are mediated by leukotrienes and PAF, synthesis of new compounds which posess leukotriene or PAF inhibitory activity, and preferably compounds which possess both inhibitory activities, as well as the identification of existing compounds which possess either or both inhibitory activities, will provide a great benefit to society in the treatment of those diseases and disorders.

## SUMMARY OF THE INVENTION

It is therefore an object of this invention to provide 2,4-diaryl-1,3-dithiolanes which both act as PAF antagonists and inhibit biosynthetic production of leukotrienes via the 5-lipoxygenase pathway.

It is another object of this invention to provide novel 2,4-diaryl-1,3-dithiolanes as a new class of potent PAF receptor antagonists which also inhibit 5-lipoxygenase.

It is another object of this invention to provide a method of using 2,4-diaryl-1,3-dithiolanes as PAF antagonists

and 5-lipoxygenase inhibitors.

It is another object of this invention to provide stereoselective processes for the preparation of optical isomers of 2,4-diaryl-1,3-dithiolanes.

It is a further object of this invention to provide a method of treating PAF and leukotriene mediated diseases by using 2,4-diaryl-1,3-dithiolanes as dual acting PAF antagonists and 5- lipoxygenase inhibitors.

According to the invention, many new 2,4-diaryl-1,3-dithiolane compounds have been synthesized. Many of the compounds have been examined in vitro, and have been shown to possess both PAF and 5-lipoxygenase inhibition activity.

Therefore, in one embodiment, the present invention provides a compound of the formula:

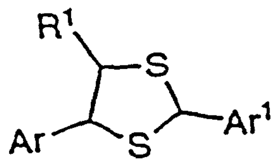

wherein $R^1$ is selected from the group consisting of hydrogen, $-CONR^2R^3$, $-COR^2$, $-CO_2R^2$, $-CH_2OR^2$, $-CH_2NR^2R^3$, $-CH_2SR^2$, and wherein Ar and $Ar^1$ are the same or different from each other and are substituted phenyl of the formula:

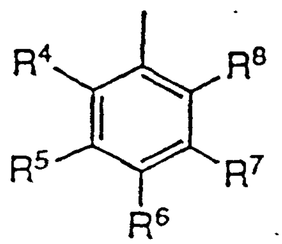

where $R^4$-$R^8$ independently are selected from the group consisting of $-NO_2$, $-NR^2R^3$, $-NR^2COR^3$, $N(OH)COR^2$, $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, halogen, $-R^2$, -CN, and $-O_2CNR^2R^3$ wherein $R^2$ and $R^3$ independently represent $C_{1-10}$ alkyl or hydrogen.

The compounds of the present invention may be employed as pharmaceuticals which include the compound and an acceptable pharmaceutical carrier. Such pharmaceutical compositions may be administered orally, topically (eg as an ointment or by means of a transdermal patch), parentally, intranasally (eg by inhalation spray), or rectally.

The term parenteral as used herein includes subcutaneous injections, intravenous, intramuscular, intrasternal injection or infusion techniques. In addition to the treatment of warm-blooded animals such as mice, rats, horses, cattle, sheep, dogs, cats, etc., the compounds of the invention are effective in the treatment of humans.

The pharamceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharamceutical compositions and such compositions may contain one or more agents selected from the group consisting of sweetening agents, flavoring agents, coloring agents and preserving agents in order to provide pharamceutically elegant and palatable preparations. Tablets contain the active ingredient in admixture with non-toxic pharamceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patent Nos. 4,256,108; 4,166,452; and 4,265,874 to form osmotic therapeutic tablets for control release.

Formulations for oral use may also be presented as hard gelatin capsules wherein the active ingredient is mixed with an inert solid diluent, for example, calcium carbonate, clacium phosphate or kaolin, or as soft gelatin capsules wherein the active ingredient is mixed with water or an oil medium, for example peanut oil, liquid paraffin, or olive

oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharamceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono or diglycerides. In addition, fatty acids such as oleic acid find use in the preparation of injectables.

The compounds may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds are employed.

Dosage levels of the order of from about 0.01 mg to about 150 mg per kilogram of body weight per day are useful in the treatment of the above-indicated conditions (about 0.7 mg to about 10 gms. per patient per day, for patients having an average body weight of 70kg). For example, inflammation may be effectively treated by the administration of from about 0.015 to about 50 mg of the compound per kilogram of body weight per day (about 1.0 mg to about 3.5 gms per patient per day). Preferably a dosage of from about 3 mg to about 20 mg per kilogram of body weight per day may produce good results (about 20 mg to about 1.5 gm per patient per day).

The amount of active ingredient that may be combined with the carrier materials to produce a single dosage form will vary depending upon the host treated and the particular mode of administration. For example, a formulation intended for the oral administration to humans may contain from 0.5 mg to 5 gm of active agent compounded with an appropriate and convenient amount of carrier material which may vary from about 5 to about 95 percent of the total composition. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular

disease undergoing therapy.

## BRIEF DESCRIPTION OF THE DRAWINGS

The foregoing and other objects, aspects and advantages will be better understood from the following detailed description of the preferred embodiments of the invention with reference to the drawings, in which:

Figure 1 is a schematic drawing showing a synthetic pathway for producing 2,4-diaryl-1,3-dithiolane compounds;
Figure 2 is a table showing the substituent groups of many 2,4-diaryl-1,3-dithiolane compounds which may be produced according to Figure 1 and the PAF and 5-lipoxygenase antagonist activities observed for several of the compounds;
Figure 3 is a schematic drawing showing a synthetic pathway for producing the oxathiolane analog of the diaryltetrahydrofuran L-652,731;
Figure 4 is a schematic drawing showing a synthetic pathway for producing a single enantiomer of a 2,4-diaryl-1,3-dithiolane compound;
Figure 5 is a schematic drawing showing the general synthetic approach for producing 2,4-diaryl-1,3-dithiolane compounds.
Figure 6 is a schematic drawing showing the synthetic approach for producing 2,4-diaryl-1,3-dithiolane compounds with an aryl amino functionality.
Figure 7 is a schematic drawing showing the synthetic approach for producing 2,4-diaryl-1,3-dithiolane compounds with an aryl hydroxyurea functionality.
Figure 8 is a schematic drawing showing the synthetic approach for producing 2,4-diaryl-1,3-dithiolane intermediates with a substituted aryl niffue or carboxylic acid functionalities.
Figure 9 is a schematic drawing showing the synthetic approach for producing 2,4-diaryl-1,3-dithiolane intermediates with a pm substituted aryl carboxylic acid or alkylthio functionalities.

The invention is also described herein with respect to the following tables, wherein:
Table 1 illustrates inhibition of PAF induced platelet aggregation and inhibition of $LTB_4$ biosynthesis by dual-acting 2,4-diaryl-1,3-dithiolanes having the following structural formula:

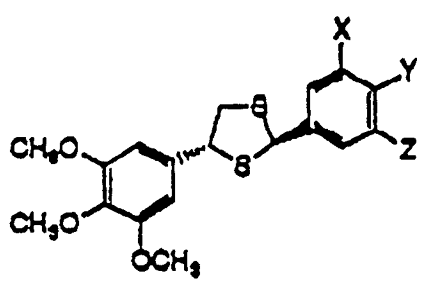

(X, Y, and Z are described in Table 1) and their cis-isomers;

## DETAILED DESCRIPTION OF THE PREFERRED EMBODIMENTS OF THE INVENTION

In vitro experiments have been conducted to determine the PAF and 5-lipoxygenase inhibitory activity of a variety of 2,4-diaryl-1,3-dithiolanes. PAF antagonist activity was determined according to the method of Shen et al., in Methods of Ezymology, Vol. 187, 447-449 (1990), and that article is herein incorporated by reference. The 5-lipoxygenase inhibitory activity was determined according to an assay discussed below.
The following outlines a platelet aggregation assay procedure. Blood is obtained from human volunteers who have not ingested aspirin or steroid drugs for ten days and is stored with a 3.8% trisodium citrate anticoagulant. Heparin is not used as the anticoagulant since it interferes with platelet aggregation. Citrated blood is centrifuged at 220 g for 10 min. at room temperature to obtain platelet-rich plasma (PRP). The platelet count is determined and is adjusted to $2.5 \times 10^7$ platelets/ml. Platelet-poor plasma (PPP) is prepared by centrifuging PRP at 1000 g for 20 min. to pelletize the platelets. PPP is used to calibrate the aggregometer. Washed platelet suspension (WPS) is prepared from PRP by underlayering the latter with Ficoll-Paque (9:2, v/v) and centrifuging at 750 g for 15 min. at room temperature. The platelets form a band between the plasma and the separation medium and are carefully collected and suspended in Tyrode's solution (in mM: NaCl, 137; KC1, 2.7; $NAHCO_3$, 11.9; $NaH_2$-$PO_4$, 0.42; $MgCl_2$, 1.0; $CaCl_2$, 1.0; HEPES, 5.0;

and dextrose, 1 g/liter, with 0.25% BSA, w/v, pH 7.4). Prostacyclin ($PGI_2$) (1 ng/ml) can be added to the suspension to prevent platelet activation; however, it should be understood that the inhibitory activity of $PGI_2$ disappears completely 10 min. from the time of addition at room temperature. The suspension is then spun at 1000 g for 10 min. and the pellet is resuspended in Tyrode's buffer for platelet aggregation. The suspensions are maintained at 37°C and stirred constantly at 1000 revolutions per min (rpm). A known amount of PAF, such as 0.11μM PAF, is added to the suspension three to five minutes after the addition of the potential PAF antagonists (the synthesized compounds). The effect of the PAF antagonist can be expressed as a percentage inhibition of the observed control maximum platelet aggregation (the aggregation observed for a suspension to which only PAF has been added) or as the concentration of the antagonist which reduces the maximum response of the platelets by 50% (commonly called the $IC_{50}$ value). To obtain the $IC_{50}$ value, varying concentrations of a potential antagonist need to be added to the test suspensions with the percentage inhibition being determined for each concentration of the antagonist, and the $IC_{50}$ value is obtained from a plot of the percentage inhibition versus concentration of the antagonist.

The 5-lipoxygenase assay can be determined according to the following procedure. Human blood is obtained from volunteers who have not taken drugs for the previous ten days. Human polymorphonuclear (PMN) leukocytes and monocytes are isolated according to the procedure by Steven Feinmark, in Methods in Enzymology, Vol. 187, 559 (1990), this article being incorporated herein by reference. Using an adapted procedure of McColl. et al., J. Chromatography, Vol. 378, 444 (1986), this article being herein incorporated by reference, the isolated PMN and/or monocyte pellets are suspended in Hank's balanced salt solution (HBSS) and cooled in an ice bath. Varying concentrations of potential antagonists (i.e., the synthesized compounds) are added to tubes containing the suspension, except for four control tubes. The suspension and potential 5-lipoxygenase inhibitors are mixed thoroughly. Arachidonic acid is added to two of the controls (0°C) followed immediately by quenching with ethyl acetate. Arachidonic acid (10μl of a 10mM solution) is then added to and mixed thoroughly with the suspension (Iml) in the remaining tubes followed by the addition of a calcium ionophore A23187 (5 μl of 1 mM solution) to all tubes except the 0°C controls (those already being quenched with ethyl acetate) and incubation of the tubes at 37°C proceeds for two minutes. The reaction in each tube was quenched and the metabolites extracted by adding ethyl acetate (1 ml) to each tube followed by centrifugation of the entire tube at 1000 g for five minutes. This extraction produces two layers where the top layer is ethyl acetate that is separated and subsequently removed by drying under a stream of nitrogen. The residues are redissolved in methanol. Leukotriene $B_4$ ($LTB_4$) is monitored using an HPLC set to absorb at 280 nm. The retention time for $LTB_4$ is determined using a pure standard, such as that which can be purchased from Sigma Chemical. Care should be taken to maintain the samples at low temperature prior to injection in the HPLC. The peak areas associated with $LTB_4$ formation are determined (preferably in duplicate and averaged) for each sample and the percent inhibition for each concentration level of inhibitor was calculated according to Equation 1:

$$\text{Eq. 1} \qquad \frac{\text{(Avg. Peak Area - Avg. Peak Area)} \atop \text{(37°C Control Antagonist)}}{\text{(Avg. Peak Area - Avg. Peak Area)} \atop \text{(37°C Control 0°C Control)}}$$

Referring now to the drawings and, more particularly to Figure 1, the novel 2,4-diaryl-1,3- dithiolanes are synthesized by first converting an arylaldehyde (A) to an epoxide (B) using tert-butylammonium iodide (TBAI), trimethylsulfonium iodide, and sodium hydroxide. The epoxide (B) is then reacted with carbon disulfide, potassium hydroxide and methanol to produce a 4-aryl-1,3-dithiolane-2-thione (C) which is then reduced with lithium aluminum hydride to produce a 1-aryl-1,2- ethanedithiol (D). The 1-aryl-1,2-ethanedithiol is then reacted with an arylaldehyde (E), which can be the same as the starting reactant, by cyclizing with pyridinium para-toluene sulfonate (PPTS) or camphor sulfonic acid. The cyclizing reaction produces 2,4- diaryl-1,3-dithiolane compounds (F) in a mixture of 1:1 to 3:1 cis:trans isomers depending on the respective catalyst. The cis and trans isomers can be obtained by successive recrystallizations in methanol, hexane/ethyl acetate, or dichloromethane/hexane.

The following examples describe the synthesis of a number of compounds which have been produced and relate particularly to Figure 1 where the intermediate compounds are identified by numbers 24- 43 and the 2,4-diaryl-1,3-dithiolane compounds produced are identified by numbers 1-21. Details on particular compounds that may also be synthesized according to the scheme of Figure 1 are also provided; however, it should be understood that compounds within the scope of this invention can be synthesized by routes other than those outlined in the following examples and that compounds with different combinations of substituents on the aryl rings are within the scope of this invention, and further that the dithiolane ring can be substituted with moieties other than hydrogen.

**EXAMPLE 1**

*Cis* and *trans*-2,4-bis-(3,4,5-trimethoxyphenyl)-1,3-dithiolane (compound 1)

Step A: Preparation of 1-(3,4,5-trimethoxyphenyl) oxirane (28)

3,4,5-trimethoxybenzaldehyde (24) (9.80g, 50.0 mmole) is dissolved in 20 ml $CH_2Cl_2$ along with tetrabutylammonium iodide (0.396g, 1.00 mmole). To this solution is added a cooled 50% NaOH solution (10g NaOH in 10 ml $H_2O$). To this mixture is added trimethysulfonium iodide (10.20g, 50.0 mmole). The reaction is refluxed with vigorous stirring for 15 hours. The reaction is quenched with $H_2O$, extracted with $CH_2Cl_2$, washed with brine, dried over $MgSO_4$ and evaporated to an oil (9.69g, 84%) which solidifies to a white solid after 24 hours in vacuo.
NMR: ($CDCl_3$) 2.75,dd,1H;3.11,dd,1H:3.81,d,1H; 3.82,s,3H;3.85,s,6H;6.50,s,2H
M.S. (CI):211 (100%). Melting Point: 54.5-56°C C,H Analysis: (theoretical, actual) C (62.85,62.63); H (6.71,6.76).

Step B: Preparation of 4-(3,4,5-trimethoxyphenyl)-1,3-dithiolane-2-thione (32)

Powdered potassium hydroxide (2.40g, 42.85 mmole) is dissolved in 10.0 ml methanol and carbon disulfide (3.10 ml, 51.43 mmole) is added at 0°C under an $N_2$ atmosphere. The reaction mixture is shaken vigorously and 1-(3,4,5-trimethoxyphenyl) oxirane (28) (3.50g, 16.66 mmole) is added. The reaction is allowed to warm to room temperature at which point the reaction starts to reflux for twenty minutes. Subsequently, the reaction is stirred at room temperature overnight. A yellow precipitate is isolated by suction filtration and is washed with $H_2O$ and diethylether to yield 3.82g (76%).
NMR: ($CDCl_3$) 3.85,s,3H;3.88,s,6H;3.99,dd,1H; 4.17,t,1H;5.57,dd,1H;6.70,s,2H.
M.S.: (IBu) 303 (100%). Melting Point: 152-154°C. C,H,S Analysis: (theoretical, actual)
C (47.66,47.90); H (4.67,4.70); S (31.80,31.71).

Step C: Preparation of 1-(3,4,5-trimethoxyphenyl)-1,2-ethanedithiol (38)

Lithium aluminum hydride (0.20g, 5.27 mmole) is added to 15 ml dry diethylether. To this slurry is added 4-(3,4,5-trimethoxyphenyl)-1,3-dithiolane-2-thione (32) (1.0g, 3.31 mmole) predissolved in 20 ml dry THF. The reaction is refluxed under an $N_2$ atmosphere for 12 hours. The reaction is cooled to 0°C and the excess hydride destroyed with $H_2O$. The reaction mixture is acidified with 10% HCl and immediately extracted with diethyl ether. The organic layer is washed with $H_2O$, dried over $MgSO_4$, filtered and evaporated to a white solid (9.836g, 97%). NMR: ($DCDl_3$) 2.34,d, 1H;2.95,m,1H;3.09,m,1H; 3.84,s,3H;3.87,s,6H;4.03,m,1H;6.53,s,2H.
M.S. (IBu) 261 (100%). Melting Point: 72.5-73.5°C C,H,S Analysis: (theoretical, actual)
C (50.74,50.85); H (6.19,6.20); S (24.63,24.53).

Step D: Preparation of *cis* and *trans*-2,4-bis-(3,4,5-trimethoxyphenyl)-1,3-dithiolane (new compound 1)

1-(3,4,5-trimethoxyphenyl)-1,2-ethanedithiol (38) (0.56g, 2.15 mmole), 3,4,5-trimethoxybenzaldehyde (24) (0.35g, 1.78 mmole) and 0.178g of pyridinium paratoluene sulfonate are added to 40 ml dry benzene and refluxed with Dean-Stark removal of the benzene-water azeotrope for 24 hours. The benzene is removed in vacuo, and the remaining oil redissolved in ethyl acetate. The organic layer is washed with $H_2O$ and was dried over $MgSO_4$, filtered, and evaporated in vacuo to a white foam which is purified by flash column chromatography using 1:1 hexane/ethyl acetate as eluent (0.630g, 81%). The trans isomer is isolated from the 1:1 mixture of diastereomers after three recrystallizations from methanol (25 mg). If the above reaction is performed under reduced pressure (aspirator) at 45°C for 2.5 hours using camphor sulfonic acid as the catalyst, the product mixture contains a 3:1 *cis*/*trans* ratio of diastereomers. The *cis* diastereomer is isolated from this product mixture by recrystallization from either methanol or hexane/ethyl acetate.
*Trans* epimer:
NMR: ($CDCl_3$): 3.48,dd,1H;3.65,dd,1H;3.84,s,6H; 3.88,s,12H;5.06,dd,1H;5.83,s,1H;6.72,s,2H;6.83,s,2H.
M.S. (CI): 439 (100%) Melting Point: 111°C C,H,S Analysis: (theoretical, actual)
C (57.51,57.57); H (5.97,5.98); S (14.62,14.52).
*Cis* epimer:
NMR: ($CDCl_3$): 3.55-3.58,d,2H;3.84-3.87,3s,18H; 4.84,t,1H;5.75,s,1H;6.75,s,2H;6.86,s,2H.
M.S. (CI): 439 (100%) Melting Point: 100-101°C C,H,S Analysis: (theoretical, actual)

C (57.51,57.40); H (5.97,6.00); S (14.62,14.68).

**EXAMPLE 2**

2-(3-hydroxy-4,5-dimethoxyphenyl)-4-(3,4,5-trimethoxyphenyl)-1,3-dithiolane (compound 2) (M.S. (CI): 425,195 (100%);

*Trans* 2-(4-hydroxy-3,5-dimethoxyphenyl)-4-(3,4,5-trimethoxyphenyl)-1, 3-dithiolane (compound 5) (C.H.S. Analysis (theoretical, actual)

C (56.58,56.68), H (5.70,5.71), S (15.10,15.04);

*Trans* 2-(4-hydroxy-3-nitro-5-methoxyphenyl)-4-(3,4,5-trimethoxyphenyl)-1,3-dithiolane (compound 6) (C.H.S. Analysis (theoretical, actual)

C (51.92,52.03), H (4.82,4.86), S (14.59,14.51);

*Trans* 2-(4-hydroxy-3-iodo-5-methoxyphenyl)-4-(3,4,5-trimethoxyphenyl)-1,3-dithiolane (compound 7) (C.H.S Analysis (theoretical,actual) C (43.85,43.93), H (4.07,4.09), S(12.32,12.42));

*Trans* 2-(4,5-dimethoxy-3-iodophenyl)-4-(3,4,5-trimethoxyphenyl)-1,3-dithiolane (compound 8) (M.S. (CI):535,195 (100%));

*Trans* 2-(4-hydroxy-3,5-dimethoxyphenyl)-4-(3,4,5-trimethoxyphenyl)-1,3-dithiolane (compound 9) (C.H.S. Analysis (theoretical, actual)

C (61.20,61.08), H (6.16,6.20), S(16.34,16.44)); and

*Trans* 2-(4-hydroxy-3,5-dimethylphenyl)-4-(3,4,5-trimethoxyphenyl)-1,3-dithiolane (compound 10) (C.H.S. Analysis (theoretical,actual)

C(65.51,65.50), H(7.61, 7.64), S(13.45,13.55)) are prepared following substantially the same procedures as described in Example 1; however, the constituents of the arylaldehyde (E) are different for each of the new compounds 2-10. Specifically, the 2,4-diaryl-1,3-dithiolanes (F) have the following constituents:

| Compound | A | B | C |
|---|---|---|---|
| 2 | OH | $OCH_3$ | $OCH_3$ |
| 5 | $OCH_3$ | OH | $OCH_3$ |
| 6 | $NO_2$ | OH | $OCH_3$ |
| 7 | I | OH | $OCH_3$ |
| 8 | I | $OCH_3$ | $OCH_3$ |
| 9 | $CH_3$ | OH | $CH_3$ |
| 10 | t-butyl | OH | t-butyl |

The aryladehydes (E) utilized are either commercially available or synthesizable by well known procedures.

**EXAMPLE 3**

*Trans* 2,4-bis-(3,5-dimethoxy-4-hydroxyphenyl)-1,3-dithiolane (compound 11) and *Trans* 2-(3,4,5-trimethoxyphenyl)-4-(3,5-dimethoxy-4-hydroxyphenyl)-1,3-dithiolane (compound 12) are prepared according to the scheme shown in Figure 1.

Step A: Preparation of 1-(3,5-dimethoxy-4-methoxymethoxyphenyl)oxirane (29)

3,5-dimethoxy-4-methoxymethoxybenzaldehyde (25) [55211-66-0] (10.85g, 48.0 mmole) is dissolved in 20 ml $CH_2Cl_2$ along with tetrabutylammonium iodide (0.40g, 1.00 mmole). To this solution is added a cooled 50% NaOH solution (10g NaOH in 10 ml $H_2O$).

To this mixture is added trimethylsulfonium iodide (10.77g, 52.81 mmole). The reaction is refluxed with vigorous stirring for 48 hours. The reaction is quenched with $H_2O$, extracted with $CH_2Cl_2$, washed with brine, dried over $MgSO_4$ and evaporated to an oil (9.69g, 84%). The oil can be crystallized from hexane/ethyl acetate. NMR: ($CDCl_3$) 2.70,m,1H; 3.10,m,1H;3.60,s,3H;3.90,s,6H;5.09,s,2H;6.50,s,2H.

Step B: Preparation of 4-(3,5-dimethoxy-4-methoxy methoxyphenyl)-1,3-dithiolane-2-thione (33)

Powdered potassium hydroxide (1.75g, 31.25 mmole) is dissolved in 3.0 ml methanol and carbon disulfide (2.85g, 37.5 mmole) was added at 0°C. The reaction mixture is shaken vigorously and 3,5-dimethoxy-4-methoxymethoxyphenyloxirane (29) (3.0g, 12.5 mmole) is added. The reaction is allowed to warm to room temperature at which point the reaction starts to reflux. The reaction is stirred at room temperature for 24 hours, and is quenched with ethyl acetate and 10% NaOH. The organic layer is washed with 3 x 40 ml 10% NaOH, 1 x 30 ml $H_2O$, 2 x 30 ml ss NaCl, dried over $MgSO_4$, and evaporated in vacuo to a yellow oil which is purified by flash column chromatography using 2:1 hexane/ethyl acetate. The product was recrystallized from hexane/ethyl acetate (4.52g, 63% yield).
NMR: ($CDCl_3$) 3.59,s,3H; 3.86,s,6H; 3.98,dd,1H; 4.16,dd,1H;5.12,s,2H;5.58,dd,1H;6.70,s,2H.
M.S.: (IBu) 333 (100%) Melting Point: 97.5-99°C C,H,S Analysis: (theoretical, actual)
C (46.97,47.06); H (4.85,4.88); s (28.93,28.83).

Step B2: Preparation of 4-(3,5-dimethoxy-4-hydroxyphenyl)-1,3-dithiolane-2-thione (34)

Powdered potassium hydroxide (3.50g, 62.50 mmole) is dissolved in 6.0 ml methanol and carbon disulfide (5.70g, 75.0 mmole) is added at 0°C. The reaction mixture is shaken vigorously and 3,5-dimethoxy-4-methoxymethoxyphenyloxirane (29) (6.0g, 25.0 mmole) is added. The reaction is allowed to warm to room temperature at which point the reaction starts to reflux. The reaction is stirred at room temperature :for 24 hours, and is quenched with ethyl acetate and 10% NaOH. The organic layer is washed with 3 x 40 ml 10% NaOH, 1 x 30 ml $H_2O$, and 2 x 30 ml ss NaCl. The ethyl acetate is reduced to 10 ml and 30 ml methanol along with 10 ml 5.0M methanolic HCl. The reaction is stirred for three hours, quenched with 100 ml $H_2O$, extracted with ethyl acetate, dried over $MgSO_4$, and evaporated in vacuo to a yellow oil which is purified by flash column chromatography using 2:1 hexane/ethyl acetate. The product is recrystallized from hexane/ethyl acetate (4.52g, 63% yield).
NMR: ($CDCl_3$) 3.91,s,6H;3.97,t,1H; 4.16,t,1H;5.58,dd,1H;5.60,s,1H;6.71,s,2H.
M.S.: (CI) 289 (100%). Melting Point: 96-97°C C,H,S Analysis: (theoretical, actual)
C (45.81,45.91); H (4.19,4.22); S(33.35,33.25).

Step C1: Preparation of 1-(3,5-dimethoxy-4-methoxymethoxyphenyl)-1,2-ethanedithiol (39)

Lithium aluminum hydride (0.622g, 16.38 mmole) is added to 60 ml dry diethyl ether. To this slurry is added dropwise 4-(3,5-dimethoxy-4-methoxymethoxy phenyl)-1,3-dithiolane-2-thione (33) (3.40g, 10.24 mmole) predissolved in 35 ml dry THF. The reaction is stirred under an $N_2$ atmosphere until the solution becomes colorless (4 hours). The reaction is cooled to 0°C and the excess hydride destroyed with $H_2O$. The reaction mixture is acidified with 10% HCl and immediately extracted with diethyl ether. The organic layer is washed with $H_2O$, dried over $MgSO_4$, filtered and evaporated to a white solid (2.47g, 84%). NMR: ($CDCl_3$) 2.33,d,1H;2.90,m,1H;3.10,m,1H; 3.59,s,3H;3.85,s,6H;4.03,m,1H;5.10,s,2H;6.53,s,2H Melting Point: 84-85°C
C,H,S analysis: (theoretical, actual)
C (49.63,49.52); H (6.25,6.28); S (22.08,22.16).

Step C2: Preparation of 1-(3,5-dimethoxy-4-hydroxyphenyl)-1,2-ethanedithiol (40)

Lithium aluminum hydride (0.553g, 14.58 mmole) is added to 10 ml dry diethyl ether and 10 ml dry THF. To this slurry is added dropwise 4-(3,5-dimethoxy-4-hydroxyphenyl) -1, 3-dithiolane-2-thione (34) (2.00g, 6.94 mmole) predissolved in 40 ml dry THF. The reaction is stirred under a nitrogen ($N_2$) atmosphere until the solution becomes colorless (1 hour). The reaction is cooled to 0°C and the excess hydride destroyed with $H_2O$. The reaction mixture is acidified with 10% HCl and immediately extracted with diethyl ether. The organic layer was washed with $H_2O$, dried over $MgSO_4$, filtered and evaporated to a white solid (1.64g, 96%).
NMR: ($CDCl_3$) 2.33,d,1H;2.93,m,1H;3.10,m,1H; 3.90,s,9H;4.03,m,1H;5.50,s,1H;6.54,s,2H.
M.S. (CI): 247,213 (100%)

Step D1: Preparation of *trans*-2,4-bis-(3,5-dimethoxy-4-hydroxyphenyl)-1,3-dithiolane (compound 11)

1-(3,5-dimethoxy-4-hydroxyphenyl-1,2-ethanedithiol (40) (2.46g, 10.0 mmole), 3,5-dimethoxy-4-hydroxybenzaldehyde (indicated as arylaldehyde E in Figure 1) (1.32g, 7.20 mmole) and 1.00g of pyridinium paratoluene sulfonate are added to 50 ml dry benzene and refluxed with Dean-Stark removal of the benzene-water azeotrope overnight. The benzene is removed in vacuo, and the remaining oil redissolved in dichloromethane. The organic layer is washed with 10% $NaHCO_3$ and $H_2O$. The organic layer is dried over $MgSO_4$, filtered, and evaporated in vacuo to a white solid which

is recrystallized from methanol (2.510g, 85%). The *trans* isomer (0.683g) is isolated by recrystallization from methanol. NMR: ($CDCl_3$) 3.45,dd,1H;3.61,dd,1H; 3.91,s,6H;3.92,s,6H;5.07,dd.1H;5.50,d,2H;5.83,s,1H; 6.73,s,2H;6.84,s,2H. M.S.: (CI) 411 (100%). Melting Point: 169-170°C C,H,S Analysis: (theoretical, actual)
C (55.59,55.58); H (5.40,5.44); S (15.62,15.56).

Step D2: Preparation of 2-(3,4,5-trimethoxyphenyl)-4-(3,5-dimethoxy-4-hydroxyphenyl)-1,3-dithiolane (compound 12)

1-(3,5-dimethoxy-4-hydroxyphenyl)-1,2-ethanedithiol (40) (2.10g, 8.54 mmole), 3,4,5-trimethoxybenzaldehyde (24) (1.28g, 6.57 mmole) and 0.857g of pyridinium paratoluene sulfonate are added to 60 ml dry benzene and refluxed with Dean-Stark removal of the benzene-water azeotrope overnight. The benzene is removed in vacuo, and the remaining oil is extracted into 10% NaOH which was washed with diethylether. The basic layer is reacidified with 10% HCl and the desired product is extracted into ethyl acetate. The organic layer is dried over $MgSO_4$, filtered, and evaporated in vacuo to an oil which is purified by flash column chromatography with 2:1 hex/ethyl acetate as eluent. The product mixture contains a 1.0/1.2 *cis*/*trans* ratio after the first recrystallization (1.287g, 59%). The *trans* isomer is isolated after six additional recrystallizations from methanol.
NMR: ($CDCl_3$) 3.50,dd,1H;3.61,dd,1H;3.84,s,3H; 3.89,s,6H;3.91,s,6H;5.06,dd,1H;5.51,s,1H;5.82,s,1H; 6.73,s,2H; 6.83,s,2H.
M.S.: (CI) 425 (100%) 212. Melting Point: 129-130°C. C,S Analysis: (theoretical, actual)
C (56.58,56.52); H (5.70,5.74).

**EXAMPLE 4**

*Trans*-2-(3,5-dithiomethyl-4-hydroxyphenyl)-1,3-dithiolane (Compound 13A (Figures 1 and 2))

3,5-dithiomethyl-4-hydroxybenzaldehyde (26) (0.822g, 3.84 mmole), which is described in European Patent Application 0,319,947, July 12, 1988, to the Green Cross Corporation and which is herein incorporated by reference, 1-(3,4,5-trimethoxyphenyl)-1,2-ethane dithiol (38) (1.20g, 4.61 mmole) and 0.460g of pyridinium paratoluene sulfonate are added to 45 ml dry benzene and refluxed with Dean-Stark removal of the benzene-water azeotrope overnight. The benzene is removed in vacuo, and the remaining oil redissolved in ethyl acetate. The organic layer is washed with 10% $NaHCO_3$ and $H_2O$. The organic layer is dried over $MgSO_4$, filtered, and evaporated in vacuo to an oil which is purified by flash column chromatography with 2:1 hex/ethyl acetate as eluent. 621 mg of product crystallize out of the column fractions as a 1.4/1.0 *trans*/*cis* mixture. (Total yield = 1.428g, 82%). The *trans* epimer is isolated as a 6/1 *trans*/*cis* mixture.
*Trans* epimer: NMR: ($CDCl_3$) 2.42,s,6H;3.47,dd,2H; 3.65,dd,2H;3.84,s,3H;3.88,s,6H;5.06,dd,1H;5.79,s,1H; 6.72,s,2H; 7.08,s,1H;7.48,s,2H.
M.S. (CI): 457 (100%) Melting Point: 144-145°C C,H,S Analysis: (theoretical, actual)
C (52.60,52.59); H (5.30,5.34); S (28.08,27.98).

2,4-bis-(3,5-dithiomethyl-4-hydroxyphenyl)-1,3-dithiolane (compound 13b in Figure 2), 2,4-bis-(3,5-dithiomethyl-4-methoxyphenyl)-1,3-dithiolane (compound 13c in Figure 2), and 2-(3,5-dithiomethyl-4-methoxyphenyl)-4-(3,5-dithiomethyl-4-hydroxyphenyl)-1,3-dithiolane (compound 13e of Figure 2) have not yet been prepared but could be prepared as described above using 3,5-dithiomethyl-4-methoxymethoxybenzaldehyde (27) as the starting material. Similarly, 2-(3,5-dithiomethyl-4-hydroxyphenyl)-4-(3,5-dithiomethyl-4-methoxyphenyl)-1,3-dithiolane (compound 13d of Figure 2) has not yet been prepared but could be prepared as described above using 3,5-dithiomethyl-4-hydroxybenzaldehyde as a starting material.

Referring now to both Figures 1 and 2, many 2,4-diaryl-1,3-dithiolane compounds have been synthesized according to the scheme shown in Figure 1 and discussed in Examples 1-4 and many other compounds are readily synthesizable via the same scheme. Several of these compounds have been tested for their antagonist activity for PAF induced platelet aggregation and for their inhibition of the production of leukotrienes via the 5-lipoxygenase pathway in PMN leukocytes and monocytes according to the procedures described above. Figure 2 shows that small concentrations of these compounds can inhibit both PAF platelet aggregation and the 5-lipoxygenase pathway; therefore, these compounds would be beneficial for the treatment of the large number of diseases and disorders which are mediated by leukotrienes and PAF.

Figure 3 shows the synthesis route for the preparation of the oxathiolane (F') analog of the diaryltetrahydrofuran L-652,731. This compound was synthesized for the purpose of comparing its antagonistic activity to compound 1 (Figures 1 and 2) of the present invention, which is the dithiolane (F) analog of diaryltetrahydrofuran L-652,731. 3,4,5-trimethoxystyrene (B') is formed by a Wittig reaction from 3,4,5-trimethoxybenzaldehyde (A') and methyltriphenylphosphoniumbromide. The styrene (B') was converted to the bromohydrin (C') using NBS and wet DMSO. The bromohydrin (C') was converted to thiouronium salt (D') using thiourea and subsequently hydrolyzed using sodium

hydroxide to produce hydroxythiol (E'). Similar to Figure 1, the hydroxythiol (E') was reacted in an acid catalyzed thioketal cyclization with 3,4,5-trimethoxy benzaldehyde to produce the oxathiolane (F'). Of particular significance, the oxathiolane (F') was found to be a much weaker antagonist of PAF-induced platelet aggregation than the corresponding dithiolane compound which had an $IC_{50}$ value of less than 1μM.

Figure 4 shows a procedure for preparing the R,S-enantiomer of compound 7 (Figures 1 and 2 show the preparation of a racemic mixture of compound 7, specifically, (R, S)-*Trans*-2-(4-hydroxy-5-iodo-3-methoxyphenyl)-4-(3,4,5-trimethoxyphenyl)-1,3-dithiolane. The importance of testing potential drugs in both their racemic and enantiomeric form has received increasing attention in the scientific community. Jamali et al., in *J. Pharm. Sci.,* 78. 695 (1989), demonstrated that the pharmokinetic and pharmodynamic interactions of a racemic drug is more complex than the sum of the contributions of the individual enantiomers. In a racemic mixture, the inactive enantiomer may agonize or antagonize pharmocologic and/or toxicologic activities. Thus, it may be preferable to use a single enantiomer for therapeutic applications. Example 5 describes the synthetic process shown in Figure 4.

**EXAMPLE 5**

(R,S)-Trans-2-(4-hydroxy-5-iodo-3-methoxyphenyl)-4-(3,4,5-trimethoxyphenyl)-1,3-dithiolane

Step A: Preparation of 2-bromo-1-(3,4,5-trimethoxyphenyl) ethanone (A'')

Freshly pulverized copper bromide (15.00g, 67.82 mmole) is refluxed in 50 ml chloroform 3,4,5-trimethoxyacetophenone (7.50g, 35.67 mmole) predissolved in 50 ml chloroform is added and the reaction refluxed overnight. The copper bromide is removed by filtration through celite and the solvent removed in vacuo to leave a dark yellow-brown oil. Crystallization of this oil has been unsuccessful and the product is purified by flash column chromatography using 2:1 hex/ethyl acetate as eluent (7.90g, 77%). 1-(3,4,5-trimethoxyphenyl)-2,2-dibromoethanone is isolated as a minor side product.
NMR: ($CDCl_3$) 3.93,s,6H;3.94,s,3H;4.41,s,2H;7.24,s,2H
Melting Point: 67-68°C

Step B: Preparation of (R)-2-bromo-1-(3,4,5-trimethoxyphenyl)ethanol (B'')

2-bromo-1-(3,4,5-trimethoxyphenyl)ethanone (Al) (7.90g, 27.33 mmole) is dissolved in 50 ml dry THF and is cooled to -20°C under an $N_2$ atmosphere. To this solution is added (-)-B-chlorodiisopino campheylborane (14.90g, 46.50 mmole) predissolved in 50 ml dry THF. The reaction is stirred at -20°C for 6 hours. THF is removed in vacuo and the remaining oil redissolved in diethyl ether. To this solution is added diethanolamine (7.33g, 69.75 mmole) and is stirred overnight. The solid is removed by filtration and the filtrate washed with $H_2O$, dried over $MgSO_4$, filtered and evaporated to an oil which is purified by flash column chromatography using 2:1 hex/ethyl acetate as eluent in order to remove the pinene side product. (6.268g, 79%: $[\alpha]^{21}D$ = -28.0° 1% $CHCl_3$). After one recrystallization from ether/hexane, the rotation increased to $[\alpha]^{21}D$ = 30.40° 1% $CHCl_3$ (5.50g, 69%).
NMR: ($CDCl_3$) 2.71,d,2H;3.51,dd,1H;3.62,dd,1H; 3.82,s,3H;3.86,s, 6H;4.85,ddd, 1H;6.60,s,2H.
M.S.: (IBu) 292,290,273. Melting Point: 79-80°C C,H,Br Analysis: (theoretical, actual)
C (45.38,45.44), H (5.19,5.22), Br (27.45,27.36).

The Mosher ester derivation of (R)-2-bromo-1-(3,4,5-trimethoxyphenyl)ethanol (C'') was prepared as follows to determine the optical purity. (R)-2-bromo-1-(3,4,5-trimethoxyphenyl)ethanol (B'') (0.025g, 0.086 mmole), (R) (+)-α-methoxy-α-(trifluoromethyl)phenylacetic acid chloride (0.024g, 0.094 mmole), dimethylaminipyridine (0.001g, 0.11 equivalents), triethylamine (0.022g, 0.215 mmole) and 5 ml dry dichloromethane are stirred at room temperature for 12 hours. The solvent is removed in vacuo, and the remaining oil redissolved in 0.025 ml ethyl acetate. This solution is added to a micro flash silica column in a disposable pasteur pipette and the product eluted with 2:1 hex/ethyl acetate (0.040g, 92%). By $^1H$-NMR, the ratio of RR/RS diasteriomers is 67/1 - 97% ee.
NMR: ($CDCl_3$) 3.57,dd,1H;3.68,dd,1H;3.69,s,6H; 3.70,d,3H;3.83,s,3H;6.04,dd,1H;6.36,s,2H;7.3-7.45,m,5H. $[\alpha]^{21}D$ = -28.4° 1% $CHCl_3$
M.S.: (IBu) 508,275,273 (base).
Melting Point: 99-101°C
C,H Analysis: (theoretical, actual) C (49.72,49.81), H (4.37,4.40).

Step C: Preparation of (S)-4-(3,4,5-trimethoxy phenyl)-1,3-dithiolane-2-thione (D'')

Lithium hydroxide monohydrate (0.6g, 1.27 mmole) is added to 8.5 ml carbon disulfide and 0.2 ml DMF and is stirred for 15 minutes under reflux. To this mixture is added (R)-2-bromo-1-(3,4,5-trimethoxy-phenyl)ethanol (B'')

(0.1g, 0.344 mmole). The reaction mixture is refluxed under an $N_2$ atmosphere for 24 hours. The solvent is removed in vacuo, and the remaining oil redissolved in ethyl acetate, washed with 10% NaOH and $H_2O$. The organic layer is dried over $MgSO_4$, filtered, and evaporated to an oil which is purified by flash column chromatography using 1:1 hexane/ethyl acetate as eluent. (0.034g, 33%: $[\alpha]^{21}D$ = + 90.5° 1% $CHCl_3$).
NMR: ($CDCl_3$) 3.85,s,3H;3.88,s,6H;3.99,dd,1H; 4.17,t,1H;5.57,dd,1H;6.70,s,2H.

Step D: Preparation of (S)-1-(3,4,5-trimethoxy phenyl)-1,2-ethanedithiol (E")

Lithium aluminum hydride (0.146g, 3.84 mmole) is added to 20 ml dry diethyl ether. To this slurry is added (s)-4-(3,4,5-trimethoxyphenyl)-1,3-dithiolane-2-thione (D") (0.725g, 2.40 mmole) predissolved in 30 ml dry THF. The reaction is stirred at room temperature under an $N_2$ atmosphere for 24 hours. The reaction is cooled to 0°C and the excess hydride destroyed with $H_2O$. The reaction mixture is acidified with 10% HCl and immediately extracted with diethyl ether. The organic layer is washed with $H_2O$, dried over $MgSO_4$, filtered and evaporated to a white solid (0.514g, 82%).
$[\alpha]^{21}D$ = +42.3" 1% $CHCl_3$.
NMR: ($CDCl_3$) 3.84,s,3H;3.87,s,6H;3.98,dd,1H; 4.13,dd,1H;5.58,dd,1H;6.69,s,2H.
The Mosher ester derivation of (S)-1-(3,4,5-trimethoxyphenyl)-1,2-ethanedithiol (F") was made as follows. (S)-1-(3,4,5-trimethoxyphenyl)-1,2-ethanedithiol (E") (0.004g, 0.015 mmole), (R)(+)-α-methoxy-α-(trifluoromethyl) phenylacetic acid chloride (0.012g, 0.04 mmole), dimethylaminopyridine (0.001g), triethylamine (0.004g, 0.04 mmole) and 4 ml dry dichloromethane are stirred at room temperature for 5 hours. The solvent is removed in vacuo, and the remaining oil redissolved in 0.5 ml ethyl acetate. This solution is added to a micro flash silica column in a disposable pasteur pipette and the product eluted with 1:1 hex/ethyl acetate (0.008g, 90%). By $^1H$-NMR, the ratio of RR/RS diastereomers is 11:1 (84% ee). NMR: ($CDCl_3$) (mixture of diastereomers) 3.2-3.4,m,2H;3.55-3.70,m,2H; 3.30, d, 3H;3.44,d, 3H;3.48,d,3H; 3.51,d,3H;3.74,s,6H;3.80,s,3H;3.81,s,6H;3.83,s,3H; 4.63,dt,2H;6.43,s,2H;6.46,s,2H;7.29-7.54,m,10H.
M.S.: (IBu) 693 (base), 443,391.

Step E: Preparation of (R,S)-2-(3-hydroxy-5-iodo-4-methoxyphenyl)-4-(3,4,5-trimethoxyphenyl)-1,3-dithiolane (G")

(S)-1-(3,4,5-trimethoxyphenyl)-1,2-ethanedithiol (E") (0.500g, 1.92 mmole]), 4-hydroxy-5-iodo-3-methoxybenzaldehyde (0.486g, 1.75 mmole) and 0.176g of pyridinium paratoluene sulfonate are added to 50 ml dry benzene and refluxed with Dean-Stark removal of the benzene-water azeotrope for 12 hours. The benzene is removed in vacuo, and the remaining oil redissolved in dichloromethane. The organic layer is washed with 10% $NaHCO_3$ and $H_2O$. The organic layer is dried over $MgSO_4$, filtered, and evaporated in vacuo to an oil which is purified by flash column chromatography with 2:1 hex/ethyl acetate as eluent. The product mixture contains a 1.0/1.2 *cis*/*trans* ratio (0.800g, 88%). The *trans* isomer is isolated after four recrystallizations from methanol (0.100g).
NMR: ($CDCl_3$) 3.49,dd,1H;3.64,dd,1H;3.84,s,3H; 3.88,s,6H;3.93,s,3H;5.05,dd,1H;5.76,s,1H;6.11,s,1H; 6.72,s,2H; 7.10,d,1H;7.50,d,1H.
M.S.: (IBu) 520 (100%) Melting Point: 145-146°C $[\alpha]^{21}D$ = + 19.5° 1% $CHCl_3$

**Example 6**

Cis and trans-2-3-nitro-4-hydroxy-5-methoxyphenyl)-4-(3,4,5--trimethoxyphe nyl)-1,3-dithiolane (1) (figure 6)

Step A Preparation of 1-(3,4,5-trimethoxyphenyl)oxirane (**37**) (figure 5)

3,4,5-trimethoxybenzaldehyde (9.80g,50.0 mmole) was dissolved in 20 ml $CH_2Cl_2$ along with tetrabutylammonium iodide (0.396g,1.00 mmole). To this solution was added a cooled 50% NaOH solution (10g NaOH in 10 ml $H_2O$). To this mixture was added trimethylsulfonium iodide (10.20g,50.0 mmole). The reaction was refluxed with vigorous stirring for 15 hours. The reaction was quenched with $H_2O$, extracted with $CH_2Cl_2$, washed with brine, dried over $MgSO_4$ and evaporated to an oil (9.69g, 84%) which solidified to a white solid after 24 hours in vacuo.
NMR ($CDCl_3$) 2.75,dd,1H; 3.11,dd,1H, 3.81,d,1H; 3.82,s,3H; 3.85,s,6H; 6.50,s,2H
M.S. (CI) 211 (100%). Melting Point 54.5-56C
Anal. Calcd. for $C_{11}H_{14}O_4$: C, 62.85; H, 6.76. Found C, 62.63; H, 6.76.

Step B Preparation of 4-(3,4,5-trimethoxyphenyl)-1,3-dithiolane-2-thione (**38**)(figure 5)

Powdered potassium hydroxide (2.40g,42.85 mmole) was dissolved in 10.0 ml methanol and carbon disulfide (3.10ml, 51.43 mmole) was added at 0°C under an $N_2$ atmosphere. The reaction mixture was shaken vigorously and

1-(3,4,5-trimethoxyphenyl) oxirane (37) (figure 5) (3.50g,16.66 mmole) was added. The reaction was allowed to warm to room temperature at which point the reaction started to reflux for twenty minutes. Subsequently, the reaction was stirred at room temperature overnight. The yellow precipitate was isolated by suction filtration and was washed with $H_2O$ and diethylether to yield 3.82g (76%).
NMR: ($CDCl_3$) 3.85,s,3H; 3.88,s,6H; 3.99,dd,1H; 4.17,t,1H; 5.57,dd,1H; 6.70,s,2H.
M.S.: (IBu) 303 (100%). Melting Point: 152-154° C.
Anal. Calcd. for $C_{12}H_{14}O_3S_3$: C, 47.66; H, 4.67: S, 31.80. Found: C, 47.90; H, 4.70; S, 31.71.

Step C Preparation of 1-(3,4,5-trimethoxyphenyl)-1,2-ethanedithiol (**39**) (figure **5**)

Lithium aluminum hydride (0.20g,5.27 mmole) was added to 15 ml dry diethyl ether. To this slurry was added 4-(3,4,5-trimethoxyphenyl)-1,3-dithiolane-2-thione **(38)** (figure **5**) (1.0g,3.31 mmole) predissolved in 20 ml dry THF. The reaction was refluxed under an $N_2$ atmosphere for 12 hours. The reaction was cooled to OC and the excess hydride destroyed with $H_2O$. The reaction mixture was acidified with 10% HCl and immediately extracted with diethyl ether. The organic layer was washed with $H_2O$, dried over $MgSO_4$, and concentrated to a white solid (0.836g, 97%).
NMR: ($CDCl_3$) 2.34,d,1H; 2.95,m,1H; 3.09,m,1H; 3.84,s,3H; 3.87,s,6H; 4.03,m,1H; 6.53,s,2H.
M.S. (IBu): 261 (100%). Melting Point: 72.5-73.5°C
Anal. Calcd. for $C_{11}H_{16}O_3S_2$: C, 50.74; H, 6.19: S, 24.63. Found: C, 50.85; H, 6.20; S, 24.53.

Step D Preparation of cis and trans 2-(4-hydroxy-3-methoxy-5-nitrophenyl)-4-(3,4,5-trimethoxyphenyl)-1,3-dithiolane (1) (figure **6**)

1-(3,4,5-trimethoxyphenyl)-1,2-ethanedithiol (**39)** (figure 5) (25.4g,97.7 mmole), 5-nitrovanillin (16.73g, 84.9 mmole), pyridinium para-toluenesulfonate (PPTS) (8.52g, 33.9 mmole) and 100 ml dry benzene were refluxed under an argon atmosphere with Dean-Stark removal of the benzene-water azeotrope for 12 hours. The reaction was cooled to room temperature and the precipitated PPTS removed by vacuum filtration. Hexane (100 ml) was added to the filtrate and the precipitate collected by vacuum filtration. The yellow solid was washed with cold water, followed by minimal cold methanol and finally cold diethyl ether to leave 28.62g of a 1:1.3 cis/trans ratio of product. Six recrystallizations from ethyl acetate/hexane gives 4.3g of pure trans diastereomer. An additional 2.4 g trans product can be obtained by repeating the above crystallization process from the material remaining in the mother liqours. Once 6.7 g of trans product was isolated, 0.315g of cis diastereomer was isolated by recrystallizing the combined mother liqours from above.
Trans epimer:
NMR ($CDCl_3$) 3.50,dd,1H; 3.66,dd,1H; 3.84,s,3H; 3.88,s,6H; 4.00,s,3H; 5.05,dd,1H; 5.80,s,1H; 6.72,s,2H; 7.41,d,1H; 7.90,d,1H.
M.S. (IBu) 440 (100%). Melting Point 149-151C.
Anal. Calcd. for $C_{19}H_{21}O_7S_2N$: C, 51.92; H, 4.82; S, 14.59. Found: C, 52.03; H, 4.86; S, 14.51.
Cis/epimer:
NMR: ($CDCl_3$) 3.53,d, 2H; 3.84,s,3H; 3.89,s,6H; 3.97,s,3H; 4.88,t,1H; 5.70,s,1H; 6.75,s,2H; 7.37,d,1H; 7.97,d,1H.
M.S. (CI) 440 (100%). Melting Point 120-121°C.
Anal. Calcd. for $C_{19}H_{21}O_7S_2N$: C, 51.92; H, 4.82; S, 14.59. Found: C, 52.05; H, 4.87; S, 3.12

**Example 7**

Preparation of Cis and trans-2-(3,4-dimethoxy-5-nitrophenyl)-4-(3,4,5-trimethoxyphenyl)-1,3-dithiolane (**2**) (figure **6**)

Using the Aldrich diazald kit, diazald (13.0g, 60.7mmole) predissolved in 100 ml diethyl ether was added dropwise to an aqueous potassium hydroxide solution (ethanol, 27 ml; $H_2O$, 21 ml; and potassium hydroxide, 13.3g) at 65°C. The diazomethane/ether distillate was added at 0°C to a solution of trans-2-(4-hydroxy-3-methoxy-5-nitrophenyl)-4-(3,4,5-trimethoxyphenyl)-1,3-dithiolane (1) (figure 6) predissolved in 25 ml chloroform and 50 ml methanol. The reaction was stirred to completely homogenize the solution and was then allowed to stand at room temperature under an argon atmosphere for 5 hours. The solvent was removed in vacuo and the remaining oil was dissolved in 10 ml diethyl ether. The product crystallized out of the ether solution to yield 3.285g (98%) of light yellow crystals.
Trans epimer
NMR: ($CDCl_3$) 3.49,dd,1H; 3.65,dd,1H; 3.84,s,3H; 3.88,s,6H; 3.96,s,3H; 3.97,s,3H; 5.04,dd,1H; 5.78,s,1H; 6.71,s,2H; 7.32,d,1H; 7.58,d,1H.
M.S. (CI) 454 (100%). Melting Point 133-134°C.
Anal. Calcd. for $C_{20}H_{23}O_7S_2N$: C, 52.97; H, 5.11; S, 14.14; N, 3.09. Found: C, 52.92; H, 5.06; S, 14.06; N, 3.14.

The cis product can be obtained in the same manner as above starting from cis-2-(4-hydroxy-3-methoxy-5-nitrophenyl)-4-(3, 4,5-trimethoxyphenyl)-1,3-dithiolane (1) (figure 6).
Cis epimer:
NMR: ($CDCl_3$) 3.55,d,2H; 3.84,s,3H; 3.88,s,6H; 3.93,s,3H; 3.97,s,3H; 4.88,t,1H; 5.70,s,1H; 6.73,s,2H; 7.30,d,1H; 7.65,d,1H.
M.S. (Cl) 454 (100%). Melting Point 99-100°C.
Anal. Calcd. for $C_{20}H_{23}O_7S_2N$: C, 52.97; H, 5.11; S, 14.14; N, 3.09. Found: C, 53.08; H, 5.09; S, 14.04; N, 3.10.

**Example 8**

Preparation of Cis and trans-2-(5-amino-3,4-dimethoxyphenyl)-4-(3,4,5-trimethoxy-phenyl)-1,3-dithiolane (3) (figure 6)

Trans-2-(3,4-dimethoxy-5-nitrophenyl)-4-(3,4,5-trimethoxyphenyl)--1,3-dithiolane (2) (figure 6) (3.10g, 6.84 mmole) was predissolved in 33 ml absolute ethanol. To this solution was added calcium chloride (0.721g, 6.50 mmole) predissolved in 7 ml $H_2O$ followed by freshly activated zinc dust (10.0g,195 mmole). The reaction was refluxed for 12 hours. The solid was removed by vacuum filtration through celite and was washed with ethyl acetate. The filtrate was washed with $H_2O$, dried over $MgSO_4$, and evaporated in vacuo to a white foam (2.52g, 87%).
Trans epimer:
NMR: ($CDCl_3$) 3.45,dd,1H; 3.63,dd,1H; 3.81,s,3H; 3.84,s,3H; 3.87,s,3H; 3.88,s,6H; 5.04,dd,1H; 5.75,s,1H; 6.56,d,1H; 6.65,d,1H; 6.72,s,2H.
M.S. (IBu): 424, 227, 195 (100%). Melting Point 48-51°C (foam).
Anal. Calcd. for $C_{20}H_{25}O_5S_2N$: C, 56.72; H, 5.95; S, 15.14; N, 3.31. Found C, 56.79; H, 5.96; S, 15.04; N, 3.35.
The cis product can be obtained in the same manner as above starting from cis-2-(3,4-dimethoxy-5-nitrophenyl)-4-(3,4,5-trimethoxyphenyl)-1,3-dithiolane (2) (figure 6).
Cis epimer:
NMR: ($CDCl_3$) 3.53,d,2H; 3.81,s,3H; 3.83,s,3H; 3.85,s,3H; 3.87,s,6H; 4.81,t,1H; 5.68,s,1H; 6.64,d,1H; 6.68,d,1H; 6.74,s,2H M.S. (Cl) 424 (100%), 227, 195, 119 .

**Example 9**

Preparation of Cis and trans-2-(3,4-dimethoxy-5-N-methylaminophenyl)-4-(3,4,5-trimethoxyphenyl)-1,3-dithiolane (**4**) (table 1)

Trans-2-(5-amino-3,4-dimethoxyphenyl)-4-(3,4,5-trimethoxyphenyl)-1,3-dithiolane (3) (figure 6) (2.07g,4.89 mmole), glacial acetic acid (57 µL, 1.0 mmole), 37% formaldehyde (75 µL, 1.0 mmole), and dry acetonitrile (30 ml) were stirred at room temperature under an argon atmosphere for 5 minutes. To this solution was added sodium cyanoborohydride (0.0698,1.1 mmole) and the reaction was stirred at room temperature for 48 hours. The precipitate was removed by filtration and the filtrate quenched with 10% potassium carbonate and extracted with dichloromethane. The organic layer was washed with brine, dried over $MgSO_4$, and concentrated in vacuo to an oil which was purified by flash column chromatography with 2:1 hex/ethyl acetate as eluent (0.400g, 91%). The excess primary amine was recovered in 92% yield.
Trans epimer:
NMR: ($CDCl_3$) 2.87,s,3H; 3.46,dd,1H; 3.65,dd,1H; 3.78,s,3H; 3.84,s,3H; 3.87,s,3H; 3.88,s,6H; 5.07,dd,1H; 5.83,s,1H; 6.51,d,1H; 6.59,d,1H; 6.73,s,2H;.
M.S. (Cl): 438 (100%).
The cis product can be obtained in the same manner as above starting from
cis-2-(5-amino-3,4-dimethoxyphenyl)-4-(3,4,5-trimethoxyphenyl)-1, 3-dithiolane (3) (figure 6).

**Example 10**

Preparation of cis and trans-2-(3,4-dimethoxy-5-N,N-dimethylaminophenyl)-4-(3,4, 5-trimethoxyphenyl)-1,3-dithiolane (**5**) (figure **6**)

Trans-2-(5-amino-3,4-dimethoxyphenyl)-4-(3,4,5-trimethoxy phenyl)-1,3-dithiolane (**3**) (figure 6) (0.031g, 0.073 mmole), 37% formaldehyde (60 uL), and sodium cyanoborohydride (0.014g,0.220 mmole) were dissolved in dry acetonitrile (2 ml). To this solution at room temperature under an argon atmosphere was added glacial acetic acid (83 µL). The reaction was stirred at room temperature for 24 hours. The precipitate was removed by filtration and the filtrate quenched with 10% potassium carbonate and extracted with dichloromethane. The organic layer was washed with

brine, dried over $MgSO_4$, and concentrated in vacuo to an oil which was purified by flash column chromatography with 2:1 hex/ethyl acetate as eluent (0.017g, 52%). The compound can be recrystallized from hexane/ethyl acetate to a white solid.
Trans epimer:
NMR: ($CDCl_3$) 2.84,s,6H; 3.46,dd,1H; 3.55,dd,1H; 3.80,s,3H; 3.84,s,3H; 3.88,s,9H; 5.07,dd,1H; 5.83,s,1H; 6.73,s,3H; 6.83,d,1H.
M.S. (CI) 452, 226, 195, 83 (100%). Melting Point 89-91°C.
Anal. Calcd. for $C_{22}H_{29}O_5S_2N$: C, 58.51; H, 6.47; S, 14.20; N, 3.10. Found: C, 58.60; H, 6.47; S, 14.13; N, 3.13.

The cis product can be obtained in the same manner as above starting from cis-2-(5-amino-3,4-dimethoxyphenyl)-4-(3,4,5-trimethoxyphenyl)-1, 3-dithiolane (**3**) (figure 6).
Cis epimer:
NMR: ($CDCl_3$) 2.83,s,6H; 3.55,d,2H; 3.79,s,3H; 3.83,s,3H; 3.87,s,9H; 4.83,t,1H; 5.73,s,1H; 6.76,bs,3H; 6.85,d,1H.
M.S. (CI): 452, 226, 117 (100%).

### Example 11

Preparation of cis and trans-N'-hydroxyl-N'-methyl-N-[2,3-dimethoxy-5-{3-(3,4,5-trimethoxyphenyl)-2,4-dithiolanyl}] phenyl urea (**6**) (figure 7)

Trans-2-(5-amino-3,4-dimethoxyphenyl)-4-(3,4,5-trimethoxyphenyl)-1,3-dithiolane (**3**) (figure 6) (0.150g, 0.355 mmole), triethylamine (50µL), triphosgene (0.035g,0.118 mmole), and 20 ml dry dichloromethane were refluxed for 2 hours under an argon atmosphere. When all of the amine had been converted to isocyanate by TLC, the reaction was cooled to room temperature and N-methyl hydroxylamine hydrochloride (0.044g,0.533 mmole) predissolved in 5 ml THF, 75 µL triethylamine, and 0.5 ml $H_2O$ was added. The reaction was stirred at room temperature overnight under an argon atmosphere. The solvent was removed in vacuo, the remaining oil redissolved in dichloromethane. The organic layer was washed with $H_2O$, dried over $MgSO_4$, and concentrated to an oil in vacuo which was purified by flash column chromatography with 2:1 hexane/ethyl acetate as eluent. (112 mg, 64% yield foam). The product can be crystallized from hexane/ethyl acetate to a white solid.
Trans epimer:
NMR: ($CDCl_3$) 3.28,s,3H; 3.45,dd,1H; 3.65,dd,1H; 3.84,s,3H; 3.85,s,3H; 3.88,s,6H; 3.90,s,3H; 5.09,dd,1H; 5.72,s,1H; 6.72,s,2H; 6.90,d,1H; 8.15,d,1H; 8.52,s,1H.
M.S. (CI): Melting Point 93-94°C.
Anal. Calcd. for $C_{22}H_{28}O_7S_2N_2$: C, 53.21; H, 5.68; S, 12.91; N, 5.66. Found: C, 53.31; H, 5.70; S, 12.84; N, 5.60.

The cis product can be obtained in the same manner as above starting from cis-2-(5-amino-3,4-dimethoxyphenyl)-4-(3,4,5-trimethoxyphenyl)-1, 3-dithiolane (3) (figure 6).
Cis epimer:
NMR: ($CDCl_3$) 3.25,s,3H; 3.55,d,2H; 3.83,s,3H; 3.84,s,3H; 3.88,s,9H; 4.84,dd,1H; 5.71,s,1H; 6.78,s,2H; 6.89,d,1H; 8.23,d,1H; 8.52,s,1H. M.S. (CI): 497, 450,424, 227, 147.

### Example 12

Preparation of cis and trans-2-(5-acetamido-3,4-dimethoxyphenyl)-4-(3,4,5-trimethoxyphenyl)-1,3-dithiolane (7) (table 1)

Trans-2-(5-amino-3,4-dimethoxyphenyl)-4-(3,4,5-trimethoxyphenyl)-1,3-dithiolane (3) (figure 6) (25 mg) was dissolved in 5 ul dry dichloromethane and cooled to OC under an argon atmosphere. To this solution was added acetic anhydride (11 µL) followed by 1 drop glacial acetic acid. The reaction was allowed to warm to room temperature overnight. The reaction was quenched with 10% $NaHCO_3$ and extracted with dichloromethane. The organic layer was dried over $MgSO_4$, and concentrated to an oil which was purified by flash column chromatography using 2:1 hexane/ethyl acetate as eluent (24 mg, 89% yield).
Trans epimer:
NMR: ($CDCl_3$) 2.21,s,3H; 3.46,dd, 1H; 3.67,dd,1H; 3.84,s,3H; 3.87,s,3H; 3.88,s,6H; 3.91,s,3H; 5.08,dd,1H; 5.82,s,1H; 6.72,s,2H; 6.97,d,1H; 7.78,bs,1H; 8.8.24,d,1H.
M.S. (CI): 466, 117 (100%).

The cis product can be obtained in the same manner as above starting from cis-2-(5-amino-3,4-dimethoxyphenyl)-4-(3,4,5-trimethoxyphenyl)-1, 3-dithiolane (3) (figure 6).

**Example 13**

Cis/trans-2-(3-methoxy-4-propoxy-5-nitrophenyl)-4-(3,4,5-trimethoxyphenyl)-1,3-dithiolane (**8**) (table 1)

Step A Preparation of 3-methoxy-5-nitro-4-propoxybenzaldehyde (**40**) (figure)

5-nitrovanillin (2.50g,12.69 mmole) and freshly pulverized potassium carbonate (5.25g,38.07 mmole) were added to 40 ml dry DMF and stirred at room temperature for 5 minutes. Propyl iodide (4.31g,25.38 mmole) was added to the reaction mixture which was stirred under an $N_2$ atmosphere at 60°C for 36 hours. The reaction mixture was quenched with 125 ml $H_2O$ and extracted with dichloromethane. The organic layer was washed with ss NaCl, dried over $MgSO_4$ and the solvent removed in vacuo to leave a yellow oil. (1.71g, 56%).
NMR: ($CDCl_3$) 1.03,t,3H; 1.81,m,2H; 3.97,s,3H; 4.21,t,2H; 7.59,d,1H; 7.80,d,1H; 9.90,s,1H.
M.S.: (IBu) 240 (100%).

Step B Preparation of cis/trans-2-(3-methoxy-4-propoxy-5-nitrophenyl)-4-(3,4,5-trimethoxyphenyl)-1,3-dithiolane (**8**) (table 1)

1-(3,4,5-trimethoxyphenyl)-1,2-ethanedithiol (**39**) (figure 5) (0.689g,2.65 mmole), 3-methoxy-5-nitro-4-propoxybenzaldehyde (**40**) (figure _) (0.633g, 2.65 mmole) and 0.266g of pyridinium para-toluenesulfonate was added to 50 ml dry benzene and refluxed with Dean-Stark removal of the benzene- water azeotrope for 24 hours. The benzene was removed in vacuo, and the remaining oil redissolved in dichloromethane and was purified by flash column chromatography with 9:1 hex/ethyl acetate as eluent. The product mixture contains a 1/1 cis/trans ratio (0.600g, 47%).
cis/trans epimers:
NMR: ($CDCl_3$) 0.98,t,6H; 1.76,m,4H; 3.46,dd,1H; 3.52,d,2H; 3.62,dd,1H; 3.81,s,6H; 3.84,s,12H; 3.98,s,3H; 3.91,s,3H; 4.05,t,4H; 4.86,t,1H; 5.03,dd,1H; 5.67,1H; 5.76,s,1H; 6.70,s,2H;
6.72,s,2H; 7.28,t,2H; 7.53,d,1H; 7.60,d,1H.
M.S. (CI): 482, 288, 268, 240 (100%), 227, 198.

**Example 14**

Preparation of cis/trans-2-(5-amino-3-methoxy-4-propoxyphenyl)-4-(3,4,5-trimethoxyphenyl)-1,3-dithiolane (**9**) (Table 1)

1:1 Cis/trans-2-(3,4-dimethoxy-5-nitrophenyl)-4-(3,4,5-trimethoxyphenyl)-1,3-dithiolane (**8**) (Table 1) (0.40g, 0.832 mmole), calcium chloride (0.088g, 0.790 mmole), freshly activated zinc dust (1.70g,33.26 mmole) and 30 ml 83% ethanol were refluxed for 4 hours. The solid was removed by vacuum filtration and the filtrate concentrated to an oil in vacuo. The oil was redissolved in dichloromethane, dried over $MgSO_4$, and purified by flash column chromatography using 2:1 hex/ethyl acetate as eluent. The product mixture (foam) contains a 1.0/1.0 cis/trans ratio (0.302g, 81%).
cis/trans epimers
NMR: ($CDCl_3$) 1.02,t,6H; 1.77,m,4H; 3.45,dd,1H; 3.53,d,2H; 3.62,dd,1H; 3.83-3.87, 4s, 24H; 3.91,t,4H; 4.81,t,1H; 5.02,dd,1H; 5.68,s,1H; 5.75,s,1H; 6.55,d,1H; 6.58,d,1H; 6.64,d,1H; 6.68,d,1H; 6.71,s,2H; 6.75,s,2H.
M.S. (CI): 452, 195 (100%).

**Example 15**

Preparation of cis/trans-2-(5-dimethylamino-3-methoxy-4-propoxyphenyl)-4-(3, 4,5-trimethoxyphenyl)-1,3-dithiolane (**10**) (Table 1)

1:1 cis/trans-2-(5-amino-3,4-dimethoxyphenyl)-4-(3, 4,5-trimethoxyphenyl)-1,3-dithiolane (**9**) (Table 1) (0.100g,0.222 mmole), 37% formaldehyde (177 uL), and sodium cyanoborohydride (0.044g,0.700 mmole) were dissolved in dry acetonitrile (4 ml). To this solution at room temperature under an argon atmosphere was added glacial acetic acid (250 μL). The reaction was stirred at room temperature for 24 hours. The precipitate was removed by filtration and the filtrate quenched with 10% potassium carbonate and extracted with dichloromethane. The organic layer was washed with brine, dried over $MgSO_4$, and concentrated in vacuo to an oil which was purified by flash column chromatography with 2:1 hex/ethyl acetate as eluent. The product mixture (oil) contains a 1.0/1.0 cis/trans ratio (0.050g, 47%).
cis/trans epimers:
NMR: ($CDCl_3$) 1.01,t,3H; 1.76,m,4H; 2.82,s,3H; 2.83,s,3H; 3.46,dd,1H; 3.62,d,2H; 3.66,dd,1H; 3.84-3.88, 4s, 24H;

4.83,t,1H; 5.07,dd,1H; 5.75,s,1H; 5.83,s,1H; 6.73,s,3H; 6.76,s,3H; 6.82,dd,1H; 6.84,dd,1H.
M.S. (Cl): 480, 195 (100%).

**Example 16**

Cis/trans-2,3-dimethoxy-5-[3-(3,4,5-trimethoxyphenyl)-2,4-dithiolanyl]phenyl carboxylic acid (11) (Table 1)

Step A Preparation of 3,4-dimethoxy-5-iodobenzaldehyde (**41**) (figure 8)

5-iodovanillin (6.0g, 21.58 mmole), potassium carbonate (5.96g, 43.16 mmole), iodomethane (9.06g, 64.77mmole) and dry DMF (50ml) were stirred at room temperature under an argon atmosphere overnight. The reaction was quenched with 250 ml $H_2O$ and extracted with diethyl ether.-The organic layer was dried over $MgSO_4$, and concentrated to an oil in vacuo which was purified by flash column chromatography using 3:1 hexane/ethyl acetate as eluent. The column gives 5.814 g (92%) of a white solid.
NMR: ($CDCl_3$) 3.92,s,6H; 7.40,s,1H; 7.84,s,1H; 9.82,s,1H.

Step B Preparation of2-(3,4-dimethoxy-5-iodophenyl)-1,3-dioxane **(42)** (figure 8)

3,4-dimethoxy-5-iodobenzaldehyde **(41)** (figure 8) (5.814g,19.9 mmole), 1,3-propanediol (6.13g, 79.6 mmole), pyridinium para-toluenesulfonate (2.0g, 7.96 mmole) and 100 ml dry benzene were refluxed with Dean-Stark removal of the benzene-water azeotrope overnight. The benzene was removed in vacuo and the remaining oil redissolved in diethyl ether and was washed with 10% $NaHCO_3$ and $H_2O$. The organic layer was washed over $MgSO_4$, and concentrated to a white solid in vacuo. (6.823g, 98%). The solid can be recrystallized from hexane/ethyl acetate.
NMR ($CDCl_3$) 1.45,d,1H; 2.20,m,1H; 3.80,s,3H; 3.87,s,3H; 3.96,dt,2H; 4.25,dd,2H; 5.40,s,1H; 7.04,d,1H; 7.46,d,1H.
M.S. (Cl) 351 (100%) Melting Point 76-77.5°C.
Anal. Calcd. for $C_{12}H_{15}O_4I$ C, 41.16; H,4.32; I, 36.24. Found C, 41.30; H, 4.33; I, 36.15.

Step C Preparation of 2,3-dimethoxy-5-formylbenzoic acid (**43**) (figure 8)

2-(3,4-dimethoxy-5-iodophenyl)-1,3-dioxane (**42**) (figure 8) (1.7g, 4.86 mmole) was dissolved in 40 ml dry THF and cooled to -78°C under an argon atmosphere. n-butyllithium ( 4.3 ml of a 1.25M soln.) was added and the reaction was warmed to OC and was stirred at that temperature for an additional 45 minutes. The aryl lithium solution was then poured onto solid carbon dioxide covered with anhydrous diethyl ether. A white precipitate formed immediately. Water was added and the organic solvent subsequently removed in vacuo. The remaining aqueous solution was extracted with ethyl acetate. The organic layer was washed with 5% sodium thiosulfate, water, and then stirred vigorously over an equivolume of 10% HCl overnight. The organic layer was separated and the product extracted into 10% $K_2CO_3$. The basic solution was acidified with 10% HCl and the product extracted into chloroform. The organic layer was dried over $MgSO_4$, and concentrated in vacuo to a white solid (0.412g, 40%). The solid can be recrystallized from hexane/ethyl acetate.
NMR: ($CDCl_3$) 3.99,s,3H; 4.19,s,3H; 7.67,s,1H;8.23,s,1H; 9.95,s,1H.
M.S. (Cl): 211 (100%) Melting Point 154-155°C.
Anal. Calcd. for $C_{10}H_{10}O_5$ : C, 57.14; H,4.80.
Found: C, 57.06; H, 4.84.

Step D Preparation of cis/trans-2,3-dimethoxy-5-[3-(3,4,5-trimethoxyphenyl)-2,4-dithiolanyl]phenyl carboxylic acid (**11**) (Table 1)

2,3-dimethoxy-5-formylbenzoic acid (**43**) (figure 8) (1.775g,8.45 mmole), 1-(3,4,5-trimethoxyphenyl)-1,2-ethanedithiol (2.51g, 9.65 mmole) (39) (figure 5), pyridinium para-toluenesulfonate (0.848g, 3.38 mmole) and 100 ml dry benzene were refluxed under an argon atmosphere with Dean-Stark removal of the benzene-water azeotrope for 7 hours. The benzene was removed in vacuo and the remaining oil purified by flash column chromatography using 2:1 ethyl acetate/hexane as eluent. The column yields 2.90g (76%, 1:1 cis/trans) of a colorless oil which titurates to a white solid in cold methanol. The solid can be recrystallized from hexane/ethyl acetate.
cis/trans epimers:
NMR: ($CDCl_3$) 3.50,dd,1H; 3.57,d,2H; 3.66,dd,1H; 3.84,s,6H; 3.88,s,12H; 3.93,s,3H; 3.97,s,3H; 4.08,s,6H; 4.87,dd, 1H; 5.08,dd,1H; 5.73,s,1H; 5.82,s,1H; 6.72,s,2H; 6.76,s,2H; 7.39,d,1H; 7.42,d,1H; 7.90,d,1H; 8.0,d,1H; 11.3,bs,1H.
M.S. (Cl): 453 (100%) Melting Point 130-132°C.
Anal. Calcd. for $C_{21}H_{24}O_7S_2$ : C, 55.74; H,5.34; S, 14.17. Found: C, 55.73; H, 5.38; S, 14.10.

**Example 17**

Preparation of cis/trans-N-hydroxyl-N-methyl-[2,3-dimethoxy-5-{3-(3,4,5-trimethoxyphenyl)-2,4-dithiolanyl}]phenyl amide (**12**) (Table 1)

1:1 cis/trans-2,3-dimethoxy-5-[3-(3,4,5-trimethoxyphenyl)-2,4-dithiolanyl]phenyl carboxylic acid (11) (Table 1) (0.650g, 1.44 mmole), dry DMF (111µL) and 20 ml dry dichloromethane was cooled to 0°C under an argon atmosphere. Oxalyl chloride (314 µL, 3.6mmole) was added and the reaction stirred for 2 hours at 0°C. The acid chloride solution was then added to a solution of N-methylhydroxylamine hydrochloride (0.481g, 5.76 mmole) predissolved in THF (17 ml), triethylamine (1.2 ml) and water (1.7 ml). The reaction was stirred at room temperature for 48 hours. The reaction was quenched with 10% HCl and extracted with dichloromethane. The organic layer was dried over $MgSO_4$, and concentrated in vacuo to an oil which was purified by flash column chromatography using 2:1 ethyl acetate/hexane as eluent. The column yields 281 mg (41%) as a white foam (1:1 cis/trans). cis/trans epimers
NMR: ($CDCl_3$) 3.24,s,6H; 3.50,dd,1H; 3.57,d,2H; 3.65,dd,1H; 3.80-3.93,5s,30H; 4.85,dd,1H; 5.05,dd,1H; 5.73,s,1H; 5.80,s,1H; 6.71,s,2H; 6.74,s,2H; 7.15,d,1H; 7.21,d,1H; 7.25,d,1H; 7.28,d,1H, 8.56,bs,1H.
M.S. (CI): 482, 391 (100%)

**Example 18**

Cis/trans-2,6-dimethoxy-4-[3-(3,4,5-trimethoxyphenyl)-2,4-dithiolanyl]phenyl carboxylic acid (**13**) (Table 1)

Step A Preparation of 2-(3,5-dimethoxyphenyl)-1,3-dioxane **(44)** (figure **9**)

3,5-dimethoxybenzaldehyde (5.00g.30.12 mmole), 1,3-propanediol (9.15g, 120.5 mmole), pyridinium para-toluenesulfonate (3.02g, 12.05 mmole) and 80 ml dry benzene were refluxed with Dean-Stark removal of the benzene-water azeotrope for 12 hours. The benzene was removed in vacuo and the remaining oil redissolved in dichloromethane and was washed with 10% $NaHCO_3$ and $H_2O$. The organic layer was washed over $Na_2SO_4$ and concentrated to an oil in vacuo which was purified by flash column chromatography using 2:1 hexane/ethyl acetate containing 0.5 % triethylamine as eluent. (5.731g, 85%).
NMR: ($CDCl_3$) 1.45,dt,1H; 2.22,m,1H; 3.79,s,6H; 3.97,dt,2H; 4.26,dd,2H; 5.43,s,1H; 6.43,t,1H; 6.65,d,2H.
M.S. (CI): 225 (100%)

Step B Preparation of 2,6-dimethoxy-4-formylbenzoic acid (**45**) (figure **9**)

Anhydrous TMEDA (2.05 ml, 13.62 mmole) and 90 ml dry THF was cooled to -78°C under an argon atmosphere. n-Butyllithium (11.35ml of a 1.20M soln.) was added and after 15 minutes, 2-(3,5-dimethoxyphenyl)-1,3-dioxane (**44**) (figure 9) (3.05g, 13.62 mmole) predissolved in 50 ml dry THF was added. The reaction was warmed to 0°C and was stirred at that temperature for an additional 60 minutes (the solution turned from a blood red color to a dark brown color). The aryl lithium solution was then poured onto solid carbon dioxide covered with anhydrous diethyl ether. A white precipitate formed immediately. When the reaction warmed to room temperature, 15 ml of 10% HCl was added and the reaction was stirred overnight. The product was then extracted into chloroform. The organic layer was dried over $MgSO_4$, and concentrated in vacuo to a white solid (1.311g, 46%). The solid can be recrystallized from hexane/ethyl acetate.
NMR: ($CDCl_3$) 3.95,s,6H; 7.11,s,2H; 9.97,s,1H.
M.S. (CI) 211 (100%) Melting Point 209-211°C.
Anal. Calcd. for $C_{10}H_{10}O_5$ : C, 57.14; H,4.80.
Found: C, 57.24; H, 4.82.

Step C Preparation of cis/trans-2,6-dimethoxy-4-[3-(3,4,5-trimethoxyphenyl)-2,4-di thiolanyl]phenyl carboxylic acid (**13**) (Table 1)

2,6-dimethoxy-4-formylbenzoic acid (**45**) (figure 9) (1.18g, 5.62 mmole), 1-(3,4,5-trimethoxyphenyl)-1,2-ethanedithiol **(39)** (figure 5) (1.68g, 6.46 mmole) , pyridinium para-toluenesulfonate (0.564g, 2.25 mmole) and 50 ml dry benzene were refluxed under an argon atmosphere with Dean-Stark removal of the benzene-water azeotrope for 8 hours. The benzene was removed in vacuo and the remaining oil purified by flash column chromatography using 2:1 ethyl acetate/hexane as eluent. The column yields 1.25g (49%, 11 cis/trans) of a white foam. The solid can be recrystallized from dichloromethane/diethyl ether.
cis/trans epimers:

NMR: ($CDCl_3$) 3.50,dd,1H; 3.57,d,2H; 3.66,dd,1H; 3.84,s,3H; 3.84,s,3H; 3.86,s,6H; 3.88,s,6H;3.91,s,6H; 3.93,s,6H; 4.88,t,1H; 5.03,dd,1H; 5.73,s,1H; 5.80,s,1H; 6.72,s,2H; 6.72,s,2H; 6.87,s,2H; 6.88,s,2H.
M.S. (CI): 453 (100%) Melting Point 124-125°C.
Anal. Calcd. for $C_{21}H_{24}O_7S_2$ : C, 55.74; H,5.34; S, 14.17. Found: C, 55.45; H, 5.41; S, 14.32.

**Example 19**

Preparation of cis/trans-N-hydroxyl-N-methyl-[2,6-dimethoxy-4-{3-(3,4,5-trimethoxyphenyl)-2,4-dithiolanyl}]phenyl amide (**14**) (table 1)

1:1 cis/trans-2,6-dimethoxy-4-[3-(3,4,5-trimethoxyphenyl)-2,4-dithiolanyllphenyl carboxylic acid (**13**) (table 1) (0.630g, 1.30 mmole), dry DMF (111µL) and 20 ml dry dichloromethane was cooled to OC under an argon atmosphere. Oxalyl chloride (314 µL, 3.6mmole) was added and the reaction stirred for 2 hours at 0°C. The acid chloride solution was then added to a solution of N-methylhydroxylamine hydrochloride (0.962g, 11.52 mmole) predissolved in THF (20 ml), triethylamine (2.4 ml) and water (2.0 ml). The reaction was stirred at room temperature overnight. The reaction was quenched with 10% HCl and extracted with chloroform. The organic layer was dried over $MgSO_4$, and concentrated in vacuo to an oil which was purified by flash column chromatography using 2:1 ethyl acetate/hexane as eluent. The column yields 335 mg (54%) as a white foam (1:1 cis/trans). cis/trans epimers
NMR: ($CDCl_3$) 3.13,s,6H; 3.45,dd,1H; 3.50,d,2H; 3.59,dd,1H; 3.3.77,s,6H; 3.77,s,6H; 3.78,s,6H; 3.79,s,6H; 3.81,s,6H; 4.81,t,1H; 5.00,dd,1H; 5.68,s,1H; 5.76,s,1H; 6.68,s,2H; 6.69,s,2H; 6.78,bs,4H.
M.S. (CI) 482, 391,89 (100%) Melting point 148-153°C
Anal. Calcd, for $C_{22}H_{27}O_7S_2N$ C, 54.87; H, 5.65; S, 13.32; N, 2.91. Found C, 54.98; H, 5.69; S, 13.21; N, 2.89.

**Example 20**

Cis and trans-2-(3,5-dimethoxy-4-methylthiophenyl)-4-(3,4,5-trimethoxyphenyl)-1,3-dithiolane (**18**) (table 1)

Step A Preparation of 2-(3,5-dimethoxy-4-methylthiophenyl)-1,3-dioxane (**47**) (figure 9)

TMEDA (898 µl,5.95 mmole) and dry THF (20 ml) were cooled to -78°C under an argon atmosphere and 1.5M n-butyl lithium (3.96 ml) was added. The solution was kept at -78°C for 15 minutes and then 2-(3,5-dimethoxyphenyl)-1,3-dioxane (**42**) (figure 8) (1.212g, 5.41 mmole) predissolved in 10 ml dry THF was added. The solution turned blood red in color after being warmed to 0°C and stirring at that temperature for 1 hour. To this solution at 0°C was added methyl disulfide (535 µl, 5.95 mmole) and the reaction was stirred for two hours and then allowed to warm to room temperature. The reaction was quenched with 10% $NaHCO_3$, and extracted with diethyl ether. The organic layer was dried over anhydrous sodium sulfate, and evaporated in vacuo to an oil which was purified by flash column chromatography using 2:1 hexane/ethyl acetate as eluent. (0.957g, 66%) (oil).
NMR: ($CDCl_3$) 1.46,dt,1H; 2.23,m,1H; 2.32,s,3H; 3.91,s,6H; 3.99,dt,2H; 4.28,dd,2H; 5.46,s,1H; 6.71,s,2H.
M.S. (CI) 271 (100%).

Step B Preparation of 3,5-dimethoxy-4-methylthiobenzaldehyde (**48**) (figure **9**)

2-(3,5-dimethoxy-4-methylthiophenyl)-1,3-dioxane (**47**) (0.945g, 3.5 mmole) was dissolved in 15 ml THF and 7 ml of 10% HCl was added. The solution was stirred overnight at room temperature. The aldehyde was extracted into diethyl ether, dried over $MgSO_4$, and concentrated in vacuo to an oil. (0.742g, 90%). NMR: ($CDCl_3$) 2.45,s,3H; 3.97,s, 6H; 7.06,s,2H; 9.92,s,1H.
M.S. (CI): 213 (100%).

Step C Preparation of cis and trans-2-(3,5-dimethoxy-4-methylthiophenyl)-4-(-3,4,5-trimethoxyphenyl)-1.3-dithiolane (**18**) (table 1)

1-(3,4,5-trimethoxyphenyl)-1,2-ethanedithiol (**39**) (0.148g,0.57 mmole), 3,5-dimethoxy-4-methylthiobenzaldehyde (**48**) (figure 9) (0.11g, 0.519 mmole) and 0.052g of pyridinium para-toluenesulfonate was added to 40 ml dry benzene and refluxed with Dean-Stark removal of the benzene- water azeotrope for 48 hours. The benzene was removed in vacuo, and the remaining oil redissolved in dichloromethane. The organic layer was washed with 3 x 30 ml $H_2O$ and was dried over $MgSO_4$, and evaporated in vacuo to an oil which was purified by flash column chromatography with 2:1 hex/ethyl acetate as eluent. The product mixture contains a 1.0/1.0 cis/trans ratio (0.151g, 64%). The trans isomer was isolated after two recrytallizations of this mixture from methanol.

trans epimer
NMR: ($CDCl_3$) 1.45,s,18H; 3.42-3.70,m,4H; 3.84,s,3H; 3.88,s,6H; 5.10,dd,1H; 5.24,s,1H; 5.88,s,1H; 6.73,s,2H; 7.38,s, 2H.
M.S. (CI): 455 (100%). Melting Point 140 - 142.5°C.
Anal. calcd. for $C_{21}H_{26}O_5S_3$: C, 55.48; H, 5.76; S, 21.16. Found: C, 55.39; H, 5.78; S, 21.08.

**Example 21**

Preparation of trans 2-(4-hydroxy-5-iodo-3-methoxyphenyl)-4-(3,4,5-trimethoxy phenel)-1,3-dithiolane (**19**) (table 1)

1-(3,4,5-trimethoxyphenyl)-1,2-ethanedithiol (**39**) (figure 5) (0.500g,1.92 mmole), 5-iodovanillin (0.411g, 1.48 mmole), pyridinium para-toluenesulfonate (0.193g, 0.769 mmole) and 50 ml dry benzene were refluxed under an argon atmosphere with Dean-Stark removal of the benzene-water azeotrope for 12 hours. The benzene was removed in vacuo, and the remaining oil redissolved in dichloromethane. The organic layer was washed with 3 x 30 ml $H_2O$ and was dried over $MgSO_4$, and evaporated in vacuo to an oil which was purified by flash column chromatography with 2: 1 hex/ethyl acetate as eluent. The product mixture contains a 1.0/1.3 cis/trans ratio (0..578g, 75%) after one crystallization from methanol. The trans isomer (50 mg) was isolated after one additional crytallization from ethanol.
Trans epimer:
NMR: ($CDCl_3$) 3.49,dd,1H; 3.64,dd,1H; 3.84,s,3H; 3.88,s,6H; 3.93,s,3H; 5.05,dd,1H; 5.76,s,1H; 6.11,s,1H; 6.72,s,2H; 7.10,d,1H; 7.50,d,1H.
M.S. (IBu ) 521 (100%). Melting Point 145-146°C.
Anal. calcd. for $C_{19}H_{21}O_5S_2I$: C, 43.85; H, 4.07; S,12.32. Found: C, 43.93; H, 4.09; S, 12.42.

**Example 22**

Preparation of cis/trans 2-(3,4-dimethoxy-5-iodophenyl)-4-(3,4,5-trimethoxyphenyl)-1, 3-dithiolane **(20)** (table 1)

1-(3,4,5-trimethoxyphenyl)-1,2-ethanedithiol (**39**) (figure 5) (1.404g,5.40 mmole), 3,4-dimethoxy-5-iodobenzaldehyde (**41**) (figure 8) (1.30g, 4.45 mmole) and 0.542g of pyridinium para-toluenesulfonate was added to 50 ml dry benzene and refluxed with Dean-Stark removal of the benzene- water azeotrope for 12 hours. The benzene was removed in vacuo, and the remaining oil redissolved in ethyl acetate. The organic layer was washed with 10% $NaHCO_3$ and $H_2O$. The organic layer was dried over $MgSO_4$, and evaporated in vacuo to an oil which was purified by flash column chromatography with 2:1 hex/ethyl acetate as eluent. The product mixture contains a 1.0/1.0 cis/trans ratio (2.38g, 100%). The trans isomer can be isolated by recrystallization from methanol.
Trans epimer:
NMR: ($CDCl_3$) 3.47,dd,1H; 3.63,dd,1H; 3.82,s,3H; 3.84,s,3H; 3.88,s,9H; 5.05,dd,1H; 5.74,s,1H; 6.72,s,2H; 7.26,d,1H; 7.56,d,1H.
M.S. (CI): 534, 501, 409, 309, 227, 195 (100%).
Melting Point 117-118°C (Recrystallized from methanol).
Anal. calcd. for $C_{20}H_{23}O_5S_2I$: C, 44.95; H, 4.34; S, 12.00. Found: C, 45.01; H, 4.35; S, 12.08.

**Example 23**

Cis/trans 2-(5-cyano-3,4-dimethoxyphenyl)-4-(3,4,5-trimethoxyphenyl)-1.3-dithiolane (**21**) (table 1)

Step A Preparation of 5-cyano-3,4-dimethoxybenzaldehyde (**49**) (figure **8**)

3,4-dimethoxy-5-iodobenzaldehyde (**41**) (figure 8) (4.40g, 15.07 mmole), copper cyanide (13.5g, 150.7 mmole) and dry DMF (45 ml) were stirred under an argon atmosphere for 18 hours at 140°C. The reaction was cooled to room temperature, filtered through celite, and the filtrate concentrated to an oil in vacuo. The dark brown oil was purified through a short flash silica column using 11 hexane/ethyl acetate as eluent to yield 2.87g (69%) of a white solid.
NMR: ($CDCl_3$) 3.96,s,3H; 4.18,s,3H; 7.61,d,1H; 7.64,d,1H; 9.87,s,1H.
M.S. (CI): 383, 192 (100%).

Step B Preparation of cis/trans 2-(5-cyano-3,4-dimethoxyphenyl)-4-(3,4,5-trimethoxyphenyl)-1,3-dithiolane (**21**)

1-(3,4,5-trimethoxyphenyl)-1,2-ethanedithiol (2.36g, 9.09 mmole), 5-cyano-3,4-dimethoxybenzaldehyde (**49**) (1.50g, 7.85 mmole) and 0.788g of pyridinium para-toluenesulfonate was added to 50 ml dry benzene and refluxed

with Dean-Stark removal of the benzene-water azeotrope for 24 hours. The benzene was removed in vacuo, and the remaining oil redissolved in ethyl acetate. The organic layer was washed with 10% $NaHCO_3$ and $H_2O$. The organic layer was dried over $MgSO_4$, and evaporated in vacuo to an oil which was purified by flash column chromatography with 2:1 hex/ethyl acetate as eluent.. The product mixture contains a 1.0/1.0 cis/trans ratio (2.91g, 86%).
Trans/cis epimers:
NMR: ($CDCl_3$) 3.49,dd,2H; 3.56.d.4H; 3.65,dd,2H; 3.84,s,6H; 3.87,s,12H; 3.89,s,3H; 3.92,s,3H; 4.02,s,6H; 4.86,t,1H; 5.04,dd,1H; 5.68,s,1H; 5.75,s,1H; 6.71,s,2H; 6.72,s,2H; 7.32,bd,2H; 7.37,d,1H; 7.42,d,1H.
M.S. (CI ) 434 (100%). Melting Point 103-106°C.
Anal. calcd. for $C_{21}H_{23}O_5NS_2$: C, 58.18; H, 5.35; S,14.79; N, 3.23. Found: C, 58.00; H, 5.38; S, 14.93; N, 3.21.

It is anticipated that the compounds of the present invention can be provided to human and animal patients in pharmaceutical doses for the purpose of inhibiting PAF and 5-lipoxygenase activity and, thereby, provide treatment or prevention of PAF and leukotriene disorders and diseases. The dose level will depend on a variety of factors including the activity of the specific compound employed and the age, sex, and physical condition of the subject being treated. It is anticipated that the dose level will be on the order of 0.1-30 mg/kg of body weight per day; however, larger and smaller amounts are anticipated. The compounds of the present invention can be administered orally, by injection, by suppository, or by any other pharmaceutically acceptable route. As is well understood in the art, the compounds can be administered as salts or the like and can be administered in conjunction with suitable inert binders, excipients, elixirs, emulsions, oils, suspensions, microencapsules, intradermal devices, ointments (topical administration), lubricating agents, and the like.

## Claims

**Claims for the following Contracting States : AT, BE, CH, Li, DE, DK, FR, GB, IT, LU, MC, NL, SE**

1. A compound of the formula:

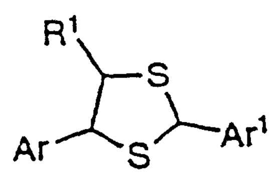

wherein $R^1$ is selected from the group consisting of hydrogen, $-CONR^2R^3$, $-COR^2$, $-CO_2R^2$, $-CH_2OR^2$, $-CH_2NR^2R^3$,-$CH_2SR^2$, and wherein Ar and $Ar^1$ are the same or different from each other and are substituted phenyl of the formula:

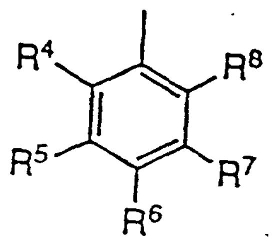

where $R^4$-$R^8$ independently are selected from the group consisting of $-NO_2$, $-NR^2R^3$, $-NR^2COR^3$, $N(OH)COR^{2'}$ $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, - $(C_5H_4N)$, halogen, $-R^2$, -CN and $-O_2CNR^2R^3$, wherein $R^2$ and $R^3$ independently represent hydrogen or $C_{1-10}$ alkyl.

2. A compound according to Claim 1 for use in medicine.

3. Use of a compound according to Claim 1 in the manufacture of a medicament for use in inhibiting PAF-induced

platelet aggregation in a patient.

**4.** Use of a compound according to Claim 1 in the manufacture of a medicament for use in inhibiting, the production of leukotrienes in a patient.

**5.** Use of a compound according to Claim 1 in the manufacture of a medicament for use in inhibiting both PAF-induced platelet aggregation and the production of leukotrienes in a patient.

**Claims for the following Contracting States : ES, GR**

**1.** A process for producing a compound of the formula:

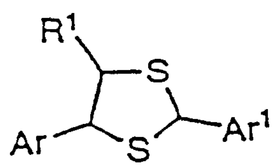

wherein $R^1$ is selected from the group consisting of hydrogen, $-CONR^2R^3$, $-COR^2$, $-CO_2R^2$, $-CH_2OR^2$, $-CH_2NR^2R^3$,-$CH_2SR^2$, and wherein Ar and $Ar^1$ are the same or different from each other and are substituted phenyl of the formula:

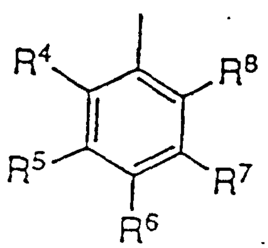

where $R^4$-$R^8$ independently are selected from the group consisting of $-NO_2$, $-NR^2R^3$, $-NR^2COR^3$, $N(OH)COR^2$, $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, halogen, $-R^2$, -CN and $-O_2CNR^2R^3$, wherein $R^2$ and $R^3$ independently represent hydrogen or $C_{1-10}$ alkyl.

**2.** A compound of the formula:

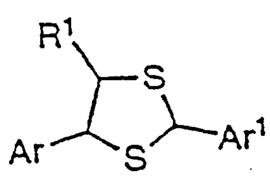

wherein $R^1$ is selected from the group consisting of hydrogen, $-CONR^2R^3$, $-COR^2$, $-CO_2R^2$, $-CH_2OR^2$, $-CH_2NR^2R^3$, $-CH_2SR^2$, and wherein Ar and $Ar^1$ are the same or different from each other and are substituted phenyl of the formula:

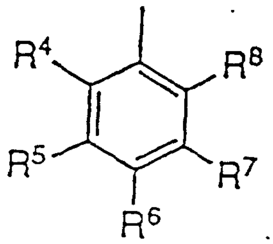

where $R^4$-$R^8$ independently are selected from the group consisting of -$NO_2$, -$NR^2R^3$, -$NR^2COR^3$, N(OH)$COR^2$, -$NR^2CONR^2R^3$, -$NR^2CON(OH)R^2$, -$CO_2R^2$, -$O_2CR^2$, -$CONR^2R^3$, -$CON(OH)R^2$, -$OR^2$, -$SR^2$, -($C_5H_4N$), halogen, -$R^2$, -CN and -$O_2CNR^2R^3$, wherein $R^2$ and $R^3$ independently represent hydrogen or $C_{1-10}$ alkyl.

**3.** A compound according to Claim 2 for use in medicine.

**4.** Use of a compound according to Claim 2 in the manufacture of a medicament for use in inhibiting PAF-induced platelet aggregation in a patient.

**5.** Use of a compound according to Claim 2 in the manufacture of a medicament for use in inhibiting, the production of leukotrienes in a patient.

**6.** Use of a compound according to Claim 2 in the manufacture of a medicament for use in inhibiting both PAF-induced platelet aggregation and the production of leukotrienes in a patient.

**Patentansprüche**

**Patentansprüche für folgende Vertragsstaaten : AT, BE, CH, Li, DE, DK, FR, GB, IT, LU, MC, NL, SE**

**1.** Verbindung mit der Formel

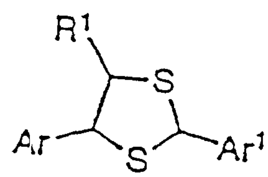

wobei $R^1$ aus einer Gruppe ausgewählt ist, die Wasserstoff, -$CONR^2R^3$, -$COR^2$, -$CO_2R^2$, -$CH_2OR^2$, -$CH_2NR^2R^3$ und -$CH_2SR^2$ umfaßt, und wobei Ar und $Ar^1$ entweder gleich oder voneinander verschieden und substituiertes Phenvl mit der Formel

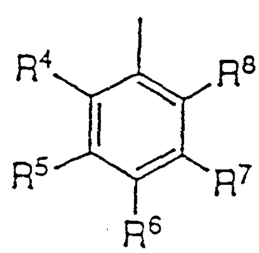

sind, wobei $R^4$ - $R^8$ unabhängig aus einer Gruppe ausgewählt sind, die -$NO_2$, -$NR^2R^3$, -$NR^2COR^3$, -N(OH)$COR^2$,

$-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, Halogen, $-R^2$, -CN und $-O_2CNR^2R^3$ umfaßt, wobei $R^2$ und $R^3$ unabhängig Wasserstoff oder ein $C_{1-10}$-Alkyl darstellen.

2. Verbindung nach Anspruch 1 zur Verwendung in der Medizin.

3. Verwendung einer Verbindung nach Anspruch 1 bei der Herstellung eines Medikaments zur Verwendung bei der Hemmung einer PAF-induzierten Plättchen-Aggregation in einem Patienten.

4. Verwendung einer Verbindung nach Anspruch 1 bei der Herstellung eines Medikaments zur Verwendung für die Hemmung der Produktion von Leukotrienen in einem Patienten.

5. Verwendung einer Verbindung nach Anspruch 1 bei der Herstellung eines Medikaments zur Verwendung bei der Hemmung sowohl der PAF-induzierten Plättchen-Aggregation als auch der Produktion von Leukotrienen in einem Patienten.

**Patentansprüche für folgende Vertragsstaaten : ES, GR**

1. Verfahren zu Herstellung einer Verbindung mit der Formel

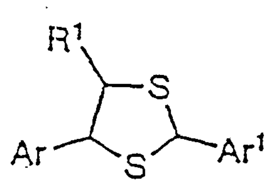

wobei $R^1$ aus einer Gruppe ausgewählt ist, die Wasserstoff, $-CONR^2R^3$, $-COR^2$, $-CO_2R^2$, $-CH_2OR^2$, $-CH_2NR^2R^3$ und $-CH_2SR^2$ umfaßt, und wobei Ar und $Ar^1$ entweder gleich oder voneinander verschieden und substituiertes Phenyl mit der Formel

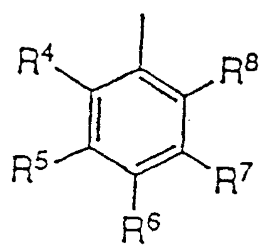

sind, wobei $R^4$ - $R^8$ unabhängig aus einer Gruppe ausgewählt sind, die $-NO_2$, $-NR^2R^3$, $-NR^2COR^3$, $-N(OH)COR^2$, $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, Halogen, $-R^2$, -CN und $-O_2CNR^2R^3$ umfaßt, wobei $R^2$ und $R^3$ unabhängig Wasserstoff oder ein $C_{1-10}$-Alkyl darstellen.

2. Verbindung mit der Formel

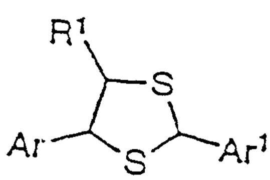

wobei $R^1$ aus einer Gruppe ausgewählt ist, die Wasserstoff, $-CONR^2R^3$, $-COR^2$, $-CO_2R^2$, $-CH_2OR^2$, $-CH_2NR^2R^3$ und $-CH_2SR^2$ umfaßt, und wobei Ar und $Ar^1$ entweder gleich oder voneinander verschieden und substituiertes

Phenyl mit der Formel

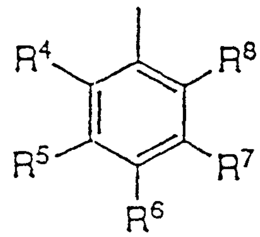

sind, wobei $R^4$ - $R^8$ unabhängig aus einer Gruppe ausgewählt sind, die $-NO_2$, $-NR^2R^3$, $-NR^2COR^3$, $-N(OH)COR^2$, $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, Halogen, $-R^2$, -CN und $-O_2CNR^2R^3$ umfaßt, wobei $R^2$ und $R^3$ unabhängig Wasserstoff oder ein $C_{1-10}$-Alkyl darstellen.

3. Verbindung nach Anspruch 2 zur Verwendung in der Medizin.

4. Verwendung einer Verbindung nach Anspruch 2 bei der Herstellung eines Medikaments zur Verwendung bei der Hemmung einer PAF-induzierten Plättchen-Aggregation in einem Patienten.

5. Verwendung einer Verbindung nach Anspruch 2 bei der Herstellung eines Medikaments zur Verwendung für die Hemmung der Produktion von Leukotrienen in einem Patienten.

6. Verwendung einer Verbindung nach Anspruch 2 bei der Herstellung eines Medikaments zur Verwendung bei der Hemmung sowohl der PAF-induzierten Plättchen-Aggregation als auch der Produktion von Leukotrienen in einem Patienten.

## Revendications

**Revendications pour les Etats contractants suivants : AT, BE, CH, Li, DE, DK, FR, GB, IT, LU, MC, NL, SE**

1. Composé de formule :

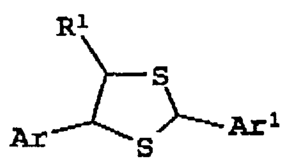

dans laquelle $R^1$ est choisi dans le groupe constitué par l'hydrogène, $-CONR^2R^3$, $-COR^2$, $-CO_2R^2$, $-CH_2OR^2$, $-CH_2NR^2R^3$, $-CH_2SR^2$, et dans laquelle Ar et $Ar^1$ sont identiques ou différents l'un de l'autre et sont un phényle substitué de formule :

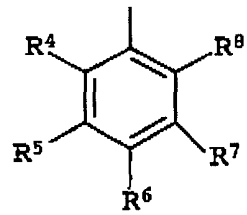

dans laquelle $R^4$ à $R^8$ sont indépendamment choisis dans le groupe constitué par $-NO_2$, $-NR^2R^3$, $-NR^2COR^3$, $N(OH)COR^2$, $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, un halogène, $-R^2$, -CN et $-O_2CNR^2R^3$, où $R^2$ et $R^3$ représentent indépendamment l'hydrogène ou un alkyle en $C_{1-10}$.

**2.** Composé selon la revendication 1 pour une utilisation en médecine.

**3.** Utilisation d'un composé selon la revendication 1 dans la fabrication d'un médicament destiné à inhiber l'agrégation plaquettaire induite par le PAF chez un patient.

**4.** Utilisation d'un composé selon la revendication 1 dans la fabrication d'un médicament pour une utilisation dans l'inhibition de la production de leucotriènes chez un patient.

**5.** Utilisation d'un composé selon la revendication 1 dans la fabrication d'un médicament pour une utilisation dans l'inhibition à la fois de l'agrégation plaquettaire induite par le PAF et la production de leucotriènes chez un patient.

**Revendications pour les Etats contractants suivants : ES, GR**

**1.** Processus de préparation d'un composé de la formule :

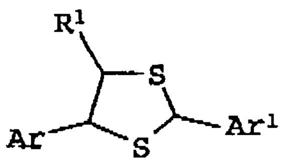

dans laquelle $R^1$ est choisi dans le groupe constitué par l'hydrogène, $-CONR^2R^3$, $-COR^2$, $-CO_2R^2$, $-CH_2OR^2$, $-CH_2NR^2R^3$, $-CH_2SR^2$, et dans laquelle Ar et $Ar^1$ sont identiques ou différents l'un de l'autre et sont un phényle substitué de formule :

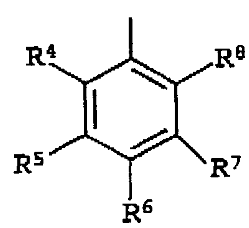

dans laquelle $R^4$ à $R^8$ sont indépendamment choisis dans le groupe constitué par $-NO_2$, $-NR^2R^3$, $-NR^2COR^3$, $N\ (OH)COR^2$, $-NR^2CONR^2R^3$, $-NR^2CON(\ OH)R^2$, $-CO_2R^2$, $-O_2CR^2$, $-CONR^2R^3$, $-CON(OH)R^2$, $-OR^2$, $-SR^2$, $-(C_5H_4N)$, un halogène, $-R^2$, -CN et $-O_2CNR^2R^3$, où $R^2$ et $R^3$ représentent indépendamment l'hydrogène ou un alkyle en $C_{1-10}$.

**2.** Composé de formule :

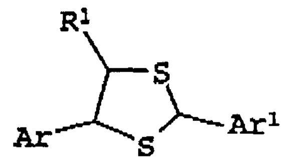

dans laquelle $R^1$ est choisi dans le groupe constitué par l'hydrogène, $-CONR^2R^3$, $-COR^2$, $-CO_2R^2$, $-CH_2OR^2$, $-CH_2NR^2R^3$, $-CH_2SR^2$, et dans laquelle Ar et $Ar^1$ sont identiques ou différents l'un de l'autre et sont un phényle substitué de formule :

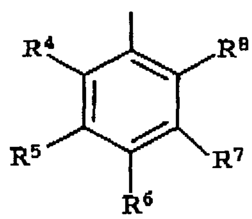

dans laquelle $R^4$ à $R^8$ sont indépendamment choisis dans le groupe constitué par $-NO_2$, $-NR^2R^3$, $-NR^2COR^3$, $N(OH)COR^2$, $-NR^2CONR^2R^3$, $-NR^2CON(OH)R^2$, $-CO_2R^2$, $-O_2CR^2$, $-CONR^2R^3$, $-CON(OH)R^2$, $-OR^2$, $-SR^2$, $-(C_5H_4N)$, un halogène, $-R^2$, -CN et $-O_2CNR^2R^3$, où $R^2$ et $R^3$ représentent indépendamment l'hydrogène ou un alkyle en $C_{1-10}$.

**3.** Composé selon la revendication 2 pour une utilisation en médecine.

**4.** Utilisation d'un composé selon la revendication 2 dans la fabrication d'un médicament pour une utilisation dans l'inhibition de l'agrégation plaquettaire induite par le PAF chez un patient.

**5.** Utilisation d'un composé selon la revendication 2 dans la fabrication d'un médicament pour une utilisation dans l'inhibition de la production de leucotriènes chez un patient.

**6.** Utilisation d'un composé selon la revendication 2 dans la fabrication d'un médicament pour une utilisation dans l'inhibition à la fois de l'agrégation plaquettaire induite par le PAF et la production de leucotrienes chez un patient.

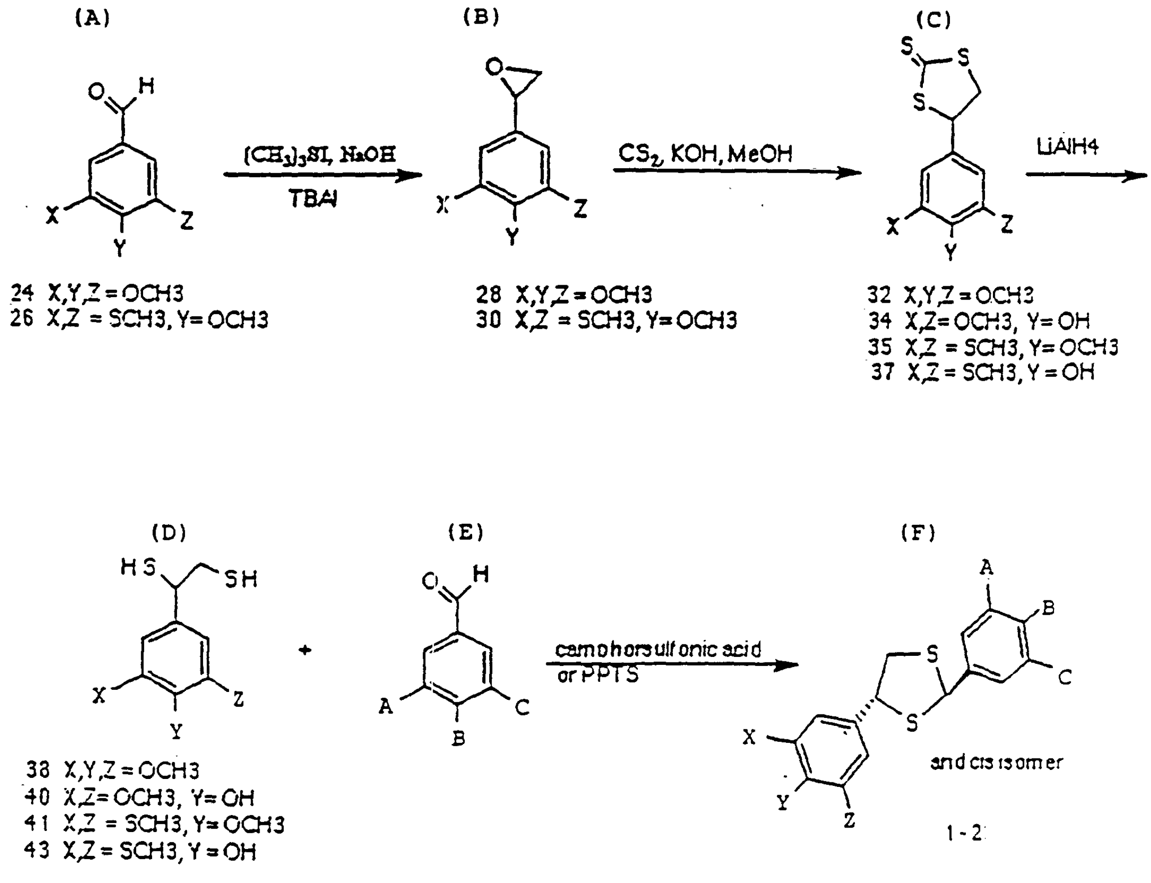
(A)
(B)
(C)
(CH3)3SI, NaOH
TBAI
CS2, KOH, MeOH
LiAlH4
24 X,Y,Z = OCH3
26 X,Z = SCH3, Y= OCH3
28 X,Y,Z = OCH3
30 X,Z = SCH3, Y= OCH3
32 X,Y,Z = OCH3
34 X,Z= OCH3, Y= OH
35 X,Z = SCH3, Y= OCH3
37 X,Z = SCH3, Y = OH
(D)
(E)
(F)
+
camphorsulfonic acid
or PPTS
38 X,Y,Z = OCH3
40 X,Z= OCH3, Y= OH
41 X,Z = SCH3, Y= OCH3
43 X,Z = SCH3, Y = OH
and cis isomer
1 - 2

Figure 1

Synthesizer Component and Inhibition of PAF Induced Platelet Aggregation and Inhibition of the Biosynthesis of LTB4

| Com # | X | Y | Z | A | B | C | PAF $IC_{50}$ | PMN $IC_{50}$ | MONO $IC_{50}$ |
|---|---|---|---|---|---|---|---|---|---|
| 1 | $OCH_3$ | $OCH_3$ | $OCH_3$ | $OCH_3$ | $OCH_3$ | $OCH_3$ | 0.8 | | |
| 2 | $OCH_3$ | $OCH_3$ | $OCH_3$ | OH | $OCH_3$ | $OCH_3$ | | | |
| 5 | $OCH_3$ | $OCH_3$ | $OCH_3$ | $OCH_3$ | OH | $OCH_3$ | 2.0 | | 8.0 |
| 6 | $OCH_3$ | $OCH_3$ | $OCH_3$ | $NO_2$ | OH | $OCH_3$ | 25.0 | | |
| 7 | $OCH_3$ | $OCH_3$ | $OCH_3$ | I | OH | $OCH_3$ | | | 30.0 |
| 8 | $OCH_3$ | $OCH_3$ | $OCH_3$ | I | $OCH_3$ | $OCH_3$ | | | |
| 9 | $OCH_3$ | $OCH_3$ | $OCH_3$ | $CH_3$ | OH | $CH_3$ | | | |
| 10 | $OCH_3$ | $OCH_3$ | $OCH_3$ | t-butyl | OH | t-butyl | | | |
| 11 | $OCH_3$ | OH | $OCH_3$ | $OCH_3$ | OH | $OCH_3$ | 15.0 | | 13.0 |
| 12 | $OCH_3$ | OH | $OCH_3$ | $OCH_3$ | $OCH_3$ | $OCH_3$ | 6.0 | | 12.0 |
| 13A | $OCH_3$ | $OCH_3$ | $OCH_3$ | $SCH_3$ | OH | $SCH_3$ | | 1.0 | 1.0 |
| 13B | $SCH_3$ | OH | $SCH_3$ | $SCH_3$ | OH | $SCH_3$ | | | |
| 13C | $SCH_3$ | $OCH_3$ | $SCH_3$ | $SCH_3$ | $OCH_3$ | $SCH_3$ | | | |
| 13D | $SCH_3$ | $OCH_3$ | $SCH_3$ | $SCH_3$ | OH | $SCH_3$ | | | |
| 13E | $SCH_3$ | OH | $SCH_3$ | $SCH_3$ | $OCH_3$ | $SCH_3$ | | | |

Figure 2

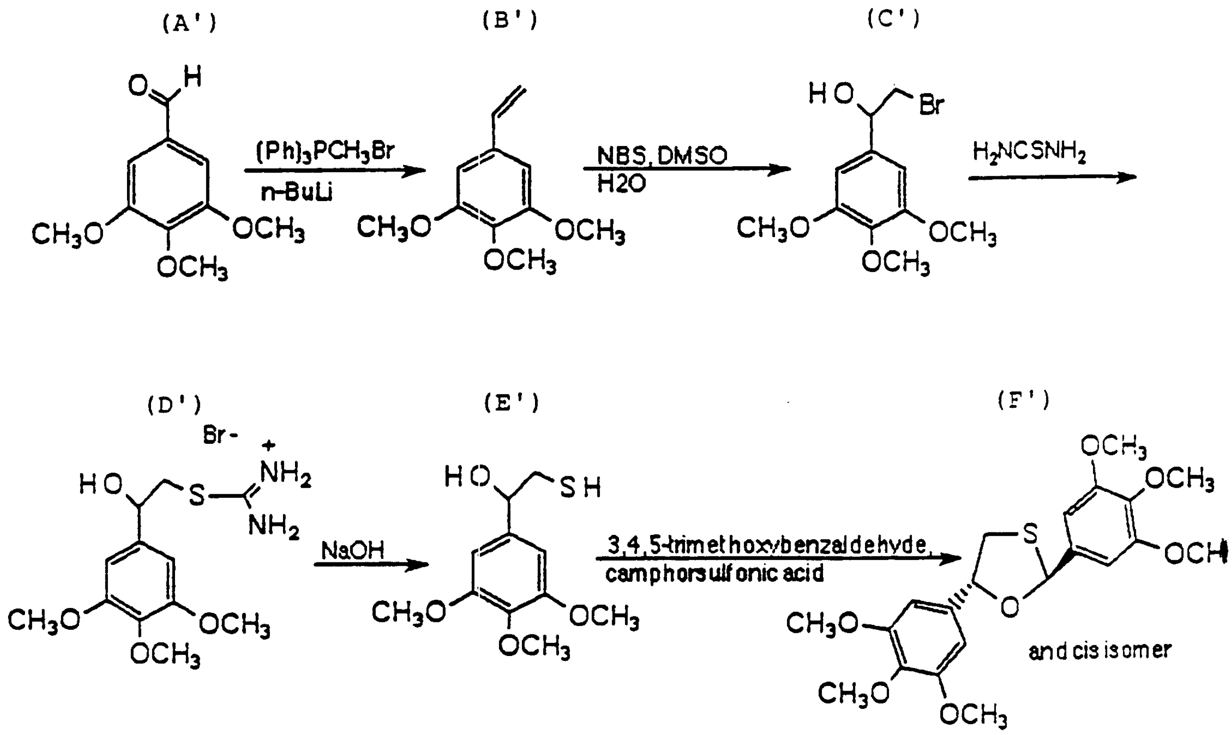
(A')
(B')
(C')
(Ph)3PCH3Br
n-BuLi
NBS, DMSO
H2O
H2NCSNH2
(D')
(E')
(F')
NaOH
3,4,5-trimethoxybenzaldehyde,
camphorsulfonic acid
and cis isomer

Figure 3

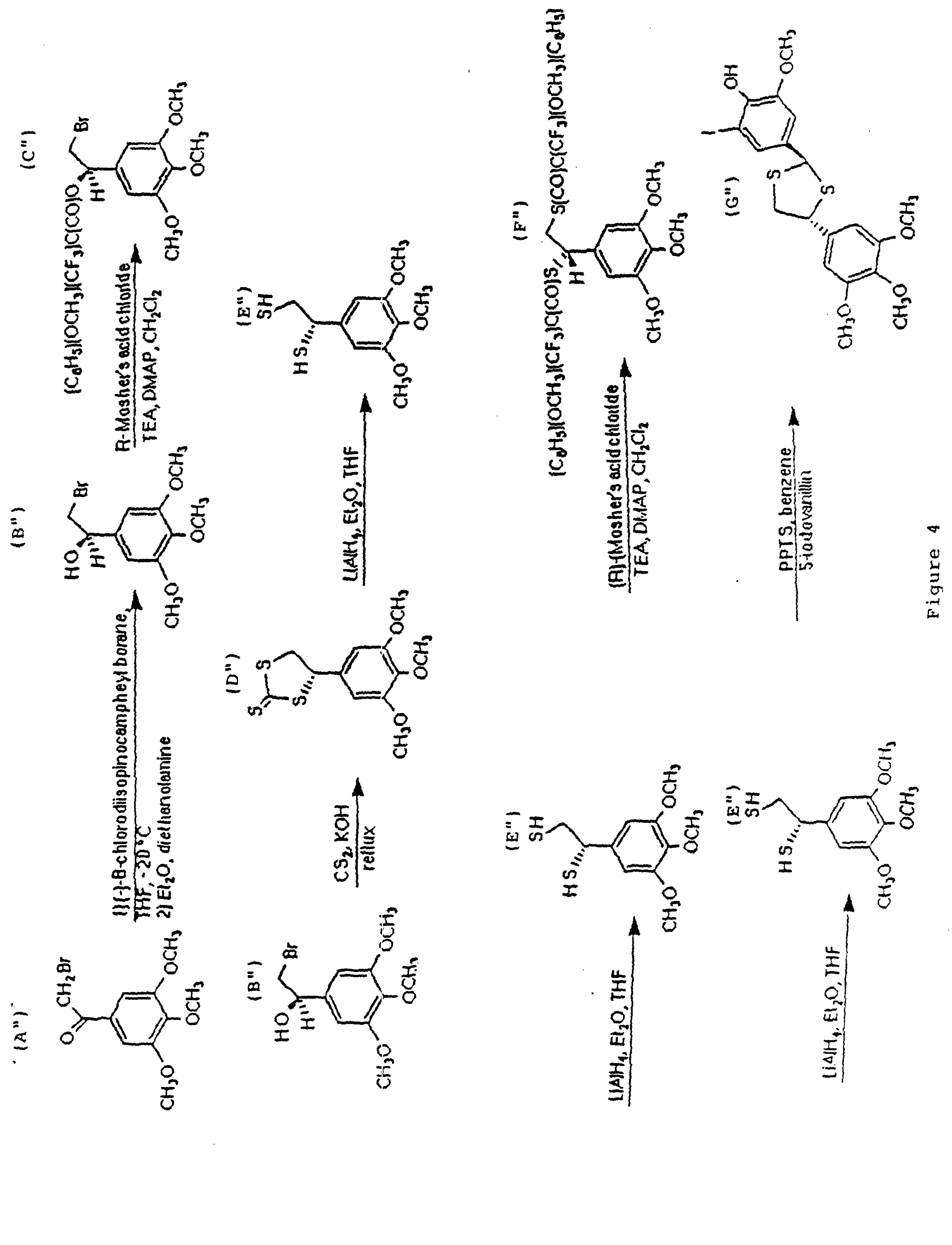
(A")
(B")
(C")
1) (-)-B-chlorodiisopinocampheyl borane, THF, -20°C
2) Et₂O, diethanolamine
R-Mosher's acid chloride
TEA, DMAP, CH₂Cl₂
(D")
(E")
CS₂, KOH
reflux
LiAlH₄, Et₂O, THF
(F")
(R)-(Mosher's acid chloride
TEA, DMAP, CH₂Cl₂
(G")
PPTS, benzene
5-iodovanillin

Figure 4

Figure 5

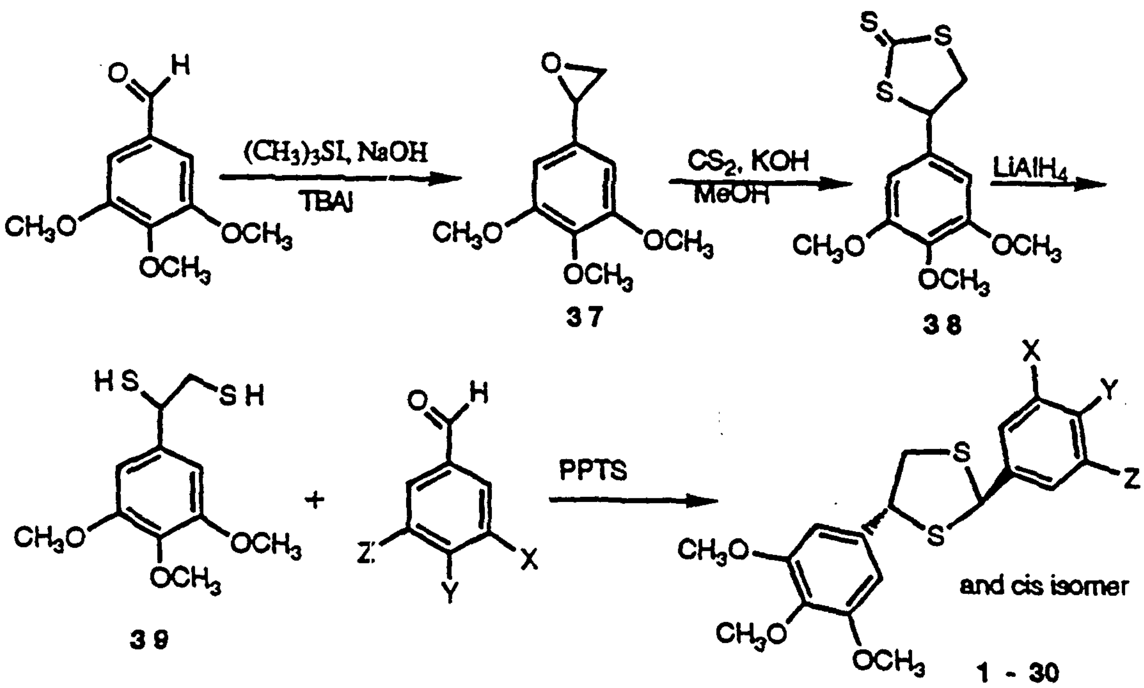


37

38

39

1 - 30

Figure 6

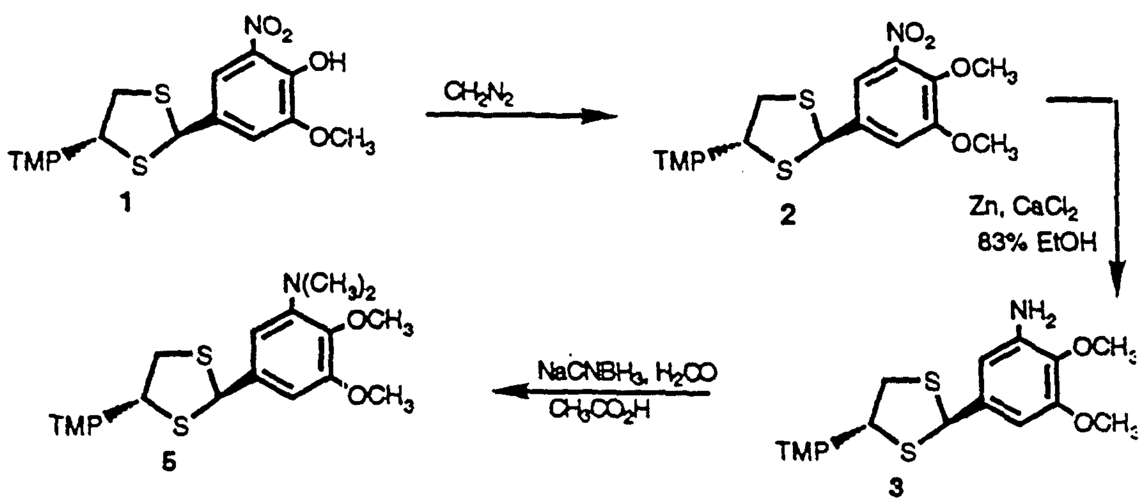


1

2

3

5

**Figure 7**

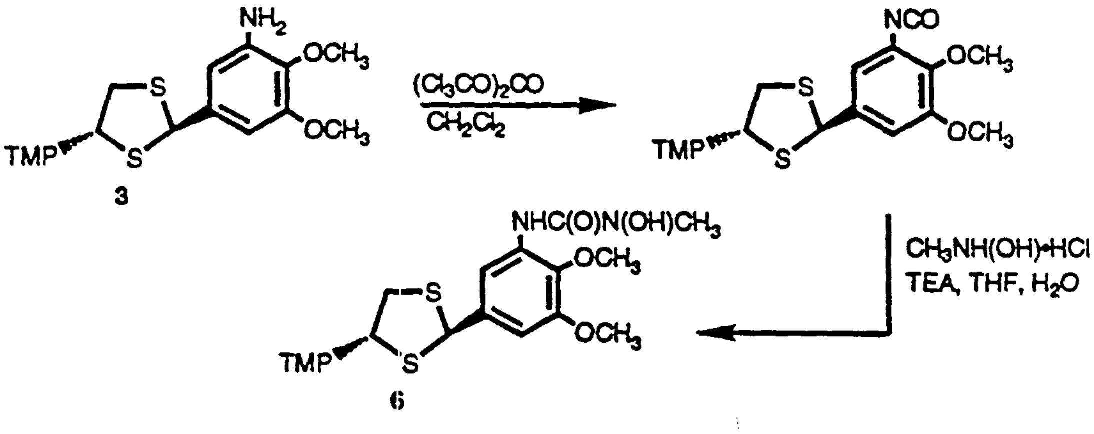


**Figure 8**

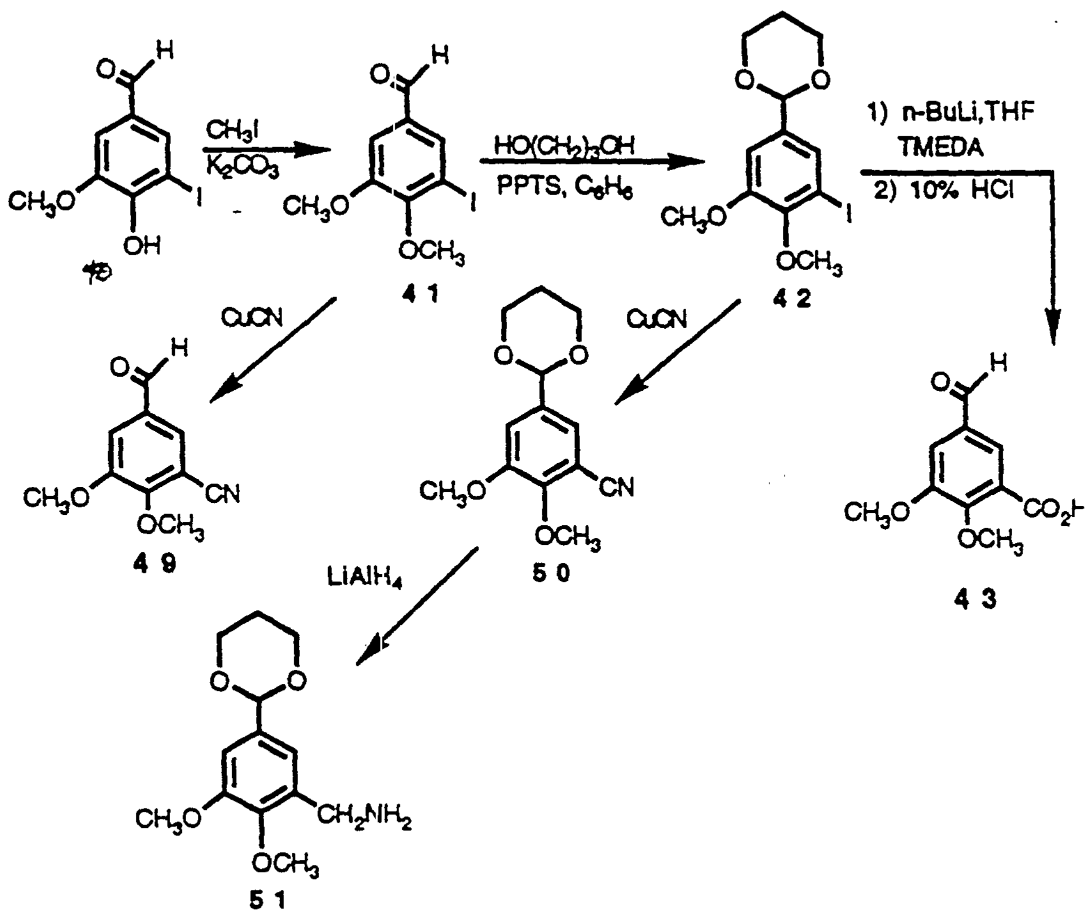

**Figure** 9

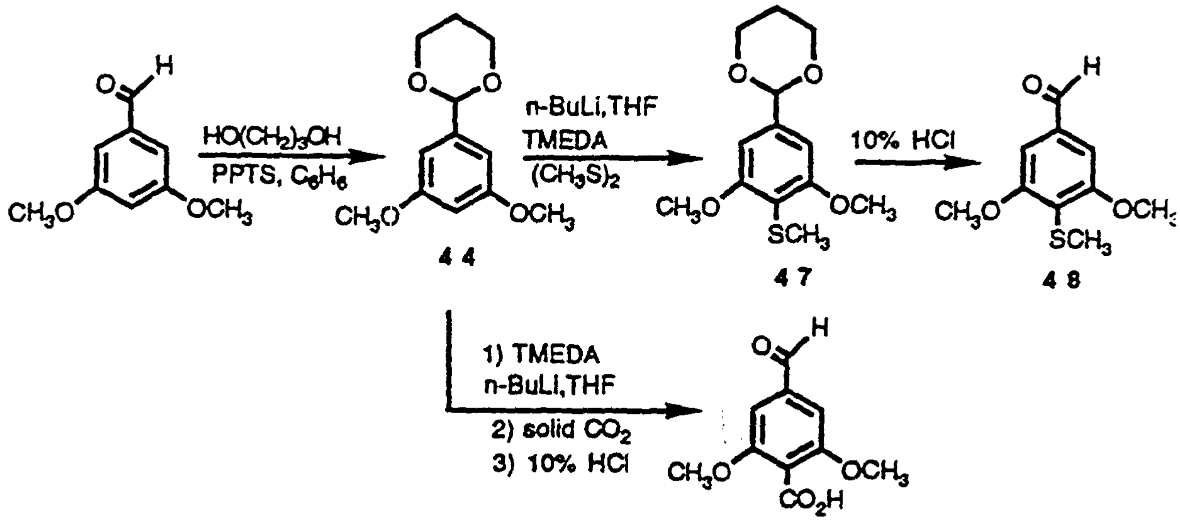


44

47

48

45

Table 1

| Cmpd # | X | Y | Z | PAF** $IC_{50}$(μM) | Monocyte*** $IC_{50}$(μM) |
|---|---|---|---|---|---|
| 1 | $NO_2$ | OH | $OCH_3$ | 8%@30μM | 7%@30μM |
| 2 | $NO_2$ | $OCH_3$ | $OCH_3$ | 14.7 | |
| 3 | $NH_2$ | $OCH_3$ | $OCH_3$ | 45.0 | |
| 4 | $NHCH_3$ | $OCH_3$ | $OCH_3$ | 4.3 | |
| 5 | $N(CH_3)_2$ | $OCH_3$ | $OCH_3$ | 0.65 | 6.7 |
| 5 Cis | $N(CH_3)_2$ | $OCH_3$ | $OCH_3$ | 14.6 | 2.3 |
| 6 | $NHCON(OH)CH_3$ | $OCH_3$ | $OCH_3$ | 5.5 | 1.9 |
| 6 Cis | $NHCON(OH)CH_3$ | $OCH_3$ | $OCH_3$ | | 0.7 |
| 7 | $NHCOCH_3$ | $OCH_3$ | $OCH_3$ | 6.6 | |
| 8* | $NO_2$ | $OCH_2CH_2CH_3$ | $OCH_3$ | 13.7 | |
| 9* | $NH_2$ | $OCH_2CH_2CH_3$ | $OCH_3$ | 15.0 | |
| 10* | $N(CH_3)_2$ | $OCH_2CH_2CH_3$ | $OCH_3$ | 6.8 | 4.1 |
| 11* | $CO_2H$ | $OCH_3$ | $OCH_3$ | | |
| 12* | $CON(OH)CH_3$ | $OCH_3$ | $OCH_3$ | 23.3 | 11.3 |
| 13* | $OCH_3$ | $CO_2H$ | $OCH_3$ | | |
| 14* | $OCH_3$ | $CON(OH)CH_3$ | $OCH_3$ | 12.8 | |
| 18 | $OCH_3$ | $SCH_3$ | $OCH_3$ | 2.0 | |
| 19 | I | OH | $OCH_3$ | 20.4 | 30.0 |
| 20 | I | $OCH_3$ | $OCH_3$ | | |
| 21 | CN | $OCH_3$ | $OCH_3$ | | |

*:Compound was tested as a 1:1 cis/trans mixture

**:L-659,989 had an $IC_{50}$ value of 0.4 μM in our human PRP PAF assay.

***:NDGA had an $IC_{50}$ value of 0.9 gM in our human monocute 5-LO assay.